# FASCICULE DE BREVET EUROPEEN

(11) **EP 2 086 996 B1**
(45) Date de publication et mention de la délivrance du brevet: **27.06.2012**
(21) Numéro de dépôt: 07822484.7
(22) Date de dépôt: 12.11.2007
(51) Int. Cl.: C07K 14/78, C07K 7/04, C07K 16/18, G01N 33/68, C07K 7/06

(54) **MARQUEUR SPECIFIQUE D'UNE DEGRADATION OXYDATIVE DE TISSUS CONTENANT DU COLLAGENE DE TYPE III, MOYENS, METHODES ET KITS DE DIAGNOSTIC, DE SUIVI OU DE PRONOSTIC DES PATHOLOGIES CIBLEES PAR CE MARQUEUR**
SPEZIFISCHER MARKER FÜR DEN OXIDATIVEN ABBAU VON GEWEBE ENTHALTEND KOLLAGEN VON TYP III, MITTEL, VERFAHREN UND KITS ZUR DIAGNOSE, ÜBERWACHUNG BZW. PROGNOSE VON PATHOLOGIEN, AUF DIE DIESER MARKER ZIELT
MARKER SPECIFIC FOR AN OXIDATIVE DEGRADATION OF TISSUES CONTAINING TYPE III COLLAGEN, MEANS, METHODS AND KITS FOR THE DIAGNOSIS, MONITORING OR PROGNOSIS OF PATHOLOGIES TARGETED BY SAID MARKER

(30) Priorité: 15.11.2006 FR 0609983
(43) Date de publication de la demande: 12.08.2009
(73) Titulaire: Synarc, 69003 Lyon (FR)
(72) Inventeur: GARNERO, Patrick, 69100 Villeurbanne (FR); CHARNI-BEN TABASSI, Nadine, 69100 Villeurbanne (FR)
(74) Mandataire: Cabinet Plasseraud
(86) Numéro de dépôt international: PCT/EP2007/062196
(87) Numéro de publication internationale: WO 2008/058922

(56) Documents cités:
- WO-A-00/79284
- WO-A-03/076946
- MEZO A R ET AL.: "Oligomerization of uniquely folded mini-protein motifs: development of a homotrimeric betabetaalpha peptide." JOURNAL OF THE AMERICAN CHEMICAL SOCIETY, vol. 123, no. 17, 2 mai 2001 (2001-05-02), pages 3885-3891, XP009086100 ISSN: 0002-7863
- YE Y Z ET AL.: "Antibodies that recognize nitrotyrosine." METHODS IN ENZYMOLOGY, vol. 269, 1996, pages 201-209, XP002041254 ISSN: 0076-6879
- REECK G R ET AL.: ""Homology" in proteins and nucleic acids: a terminology muddle and a way out of it." CELL, vol. 50, no. 5, 28 août 1987 (1987-08-28), page 667, XP002322075 ISSN: 0092-8674
- CHARNI N ET AL.: "P94: NITROSYLATED N-TELOPEPTIDE OF TYPE III COLLAGEN (IIINYS): A NEW SPECIFIC BIOCHEMICAL MARKER OF OXIDATIVE-INDUCED SYNOVIAL TISSUE METABOLISM IN ARTHRITIS" OSTEOARTHRITIS AND CARTILAGE, BAILLIERE TINDALL, vol. 14, no. Suppl2, 7 décembre 2006 (2006-12-07), - 10 décembre 2006 (2006-12-10) page S62, XP005831627 LONDON, GB ISSN: 1063-4584
- RICHARDOT P ET AL.: "116 NITRIC OXIDE-INDUCED ALTERATIONS OF SYNOVIAL TISSUE IN KNEE OSTEOARTHRITIS AND RHEUMATOID ARTHRITIS IS REFLECTED BY INCREASED JOINT FLUID AND CIRCULATING LEVELS OF NITROSYLATED N-TELOPEPTIDE OF TYPE III COLLAGEN (IIINYS)" OSTEOARTHRITIS AND CARTILAGE, BAILLIERE TINDALL, vol. 15, 6 décembre 2007 (2007-12-06), - 9 décembre 2007 (2007-12-09) pages C73-C74, XP022395887 LONDON, GB ISSN: 1063-4584

## Description

### DOMAINE TECHNIQUE

Le domaine de l'invention est celui des outils et des méthodes de diagnostic, suivi et pronostic de maladies qui s'accompagnent d'une dégradation oxydative de tissus contenant du collagène de type III. Il peut s'agir notamment de maladies ostéo-articulaires telles que sont notamment l'arthrose, la polyarthrite rhumatoïde, l'arthrite psoriasique, la goutte, la chondrocalcinose, l'arthrite induite par Yersenia, l'arthrite pyrophosphate, l'arthrite septique ou les désordres liés aux disques intervertébraux comme les spondylarthrites. Plus précisément, l'invention concerne les moyens et les procédés d'évaluation de la dégradation du tissu synovial, en particulier du collagène de type III synovial, sous l'effet d'un stress oxydatif propre à ces maladies, notamment les maladies ostéo-articulaires.

Plus précisément encore, l'invention vise en tant que produits *per se,* des marqueurs biologiques peptidiques, les éléments de reconnaissance de ces marqueurs (anticorps), les procédés et les kits de détection desdits marqueurs à l'aide desdits éléments, en vue d'applications du type diagnostic, suivi et pronostic des maladies susvisées, notamment les maladies ostéo-articulaires.

L'invention est également relative au moyen d'obtention des marqueurs peptidiques et des anticorps de reconnaissance desdits marqueurs, ces moyens étant par exemple des séquences nucléiques.

### ART ANTERIEUR- POSITION DU PROBLEME

Le diagnostic, le traitement, le suivi et le pronostic de maladies qui s'accompagnent d'une dégradation oxydative de tissus contenant du collagène de type III, est un enjeu majeur de santé publique.

Comme exemples de ces maladies, on peut citer les cancers, les maladies neurodégénératives tels que la maladie de Parkinson ou la maladie d'Alzeimer....

Le collagène de type III est un homotrimère de chaînes de collagène identique alpha 1 (III), bordées par des régions linéaires télopeptidiques. Le collagène de type III est localisé avec le collagène de type I dans la peau et les tissus vasculaires. Le collagène de type III est le collagène fibrillaire majeur de la peau et du tissu vasculaire. Au niveau de l'articulation, le collagène de type III est le collagène majoritaire de la synoviale.

Les affections inflammatoires telles que les maladies ostéo-articulaires, représentent une part significative de ce type de maladies qui s'accompagnent d'une dégradation oxydative de tissus contenant du collagène de type III.

L'arthrose et la polyarthrite rhumatoïde (arthrite) sont deux maladies ostéo-articulaires parmi les plus communes.

L'arthrose peut être due à l'âge ou à un traumatisme. L'arthrose provoque des lésions sévères au niveau du cartilage, une immobilité et des excroissances osseuses au niveau de l'articulation (ostéophytes). Cette maladie touche l'articulation entière, ce qui englobe le cartilage, la synoviale et les os reliés l'un à l'autre par cette articulation. La dégradation du cartilage dans l'arthrose est caractérisée par deux phases : une phase de biosynthèse pendant laquelle les chondrocytes tentent de réparer la matrice extracellulaire endommagée et une phase de dégradation où les enzymes produites par les chondrocytes digèrent la matrice. L'activité de biosynthèse est alors incapable de faire face à l'activité catabolique, ce qui conduit à une inhibition de la synthèse de la matrice et à une accélération de l'érosion du cartilage. L'arthrose est donc une exagération du processus de remodelage osseux dans l'articulation. Dans le cartilage normal adulte, les chondrocytes synthétisent les constituants de la matrice lentement. Le renouvellement de la matrice est strictement régulé : il y a un équilibre entre la synthèse et la dégradation.

La polyarthrite rhumatoïde est une maladie auto-immune provoquant une inflammation chronique au niveau des articulations et une destruction du cartilage, des os, des tendons et des ligaments. L'inflammation a pour origine une défaillance du système immunitaire qui ne reconnaît pas les cellules du soi et les détruit. Dans la polyarthrite rhumatoïde, l'inflammation de la synoviale provoque une production excessive de liquide synovial et d'enzymes par les synoviocytes. Celles-ci sont à l'origine de la dégradation du cartilage et de l'os. Lorsque cette situation devient chronique, il en résulte une inflammation, une douleur, et, éventuellement, un problème de mobilité.

Il est difficile de diagnostiquer ces maladies rapidement. En effet, la douleur et la raideur de l'articulation n'apparaissent que tardivement et la radiographie ne permet de détecter la maladie qu'à un stade avancé de celle-ci. Il existe donc un intérêt majeur à trouver des marqueurs spécifiques (par exemple sérique ou urinaire) du renouvellement des constituants de l'articulation. L'utilisation de ces marqueurs biologiques spécifiques est d'autant plus intéressante qu'elle constitue une technique non invasive qui permet de détecter précocement la maladie, de suivre son évolution et d'établir un pronostic à son égard.

Les marqueurs biologiques ou biochimiques du renouvellement articulaire peuvent être des marqueurs osseux spécifiques de la formation osseuse ou de la résorption osseuse, des marqueurs cartilagineux spécifiques de la formation du cartilage ou de la dégradation du cartilage, ou bien encore des marqueurs de la synoviale spécifique de la synthèse ou de la dégradation de cette dernière.

Dans le cadre de la présente invention, on s'intéresse plus particulièrement aux marqueurs biologiques ou biochimiques de la synoviale.

Par ailleurs, il est connu que l'oxydation des protéines par des espèces hyper-oxydantes, est un phénomène impliqué dans la pathogenèse de nombreuses maladies telles que les maladies neurodégénératives, les cancers, l'artériosclérose, la cataracte, la dysplasie, la dystrophie et les maladies inflammatoires dont notamment les maladies ostéo-articulaires ainsi que dans le vieillissement.

Parmi les espèces hyper-oxydantes, figure le monoxyde d'azote (NO). NO a une fonction de régulation mais exerce également, lorsqu'il est surexprimé dans des conditions inflammatoires, un effet cytotoxique induisant un stress oxydatif. Dans sa fonction de régulation, il agit comme un agent protecteur qui supprime la prolifération des cellules et la synthèse protéique. Il permet ainsi d'atténuer la réaction inflammatoire. Mais, NO peut également jouer le rôle d'agent de stress oxydatif en inhibant la régénération des tissus et la synthèse de nouvelles protéines matricielles.

Le pouvoir hyper-oxydant de NO et de ses sous-produits de réaction avec l'anion super oxyde O₂⁻, est à l'origine de sa cytotoxicité.

Dans le cas de maladies ostéo-articulaires du type polyarthrite rhumatoïde ou arthrose, la surexpression de NO par les chondrocytes, les macrophages et le tissu synovial en inflammation, est significative mais n'est pas spécifique des pathologies articulaires.

On sait en outre que le stress oxydatif induit par NO et ses sous-produits peroxydants, a notamment pour effet une nitrolysation (ou nitration), des composés aromatiques tels que les acides aminés aromatiques que sont la phénylalanine, la tyrosine et le tryptophane, qui sont présents dans de nombreux peptides et protéines, en particulier au niveau des articulations. Une protéine articulaire bien connue est le collagène de type, I, II, III, VI, IX ou XI.

Il a donc été imaginé de choisir la nitrotyrosine comme marqueur d'un stress oxydatif associé aux maladies entraînant des dommages dus à l'oxydation.

Un anticorps spécifique des tyrosines nitrosylées a été décrit dans un article scientifique de Ze Y Z et al. 1996, Methods in enzymology, vol. 269 : pp 201-209).

La demande de brevet PCT WO-A-96/04311 décrit ainsi des anticorps anti-nitrotyrosine aptes à reconnaître et à se lier à la nitrotyrosine et à des résidus de nitrotyrosine au sein de protéines. Ces anticorps anti-nitrotyrosine sont décrits comme utiles dans des méthodes pour détecter diverses conditions pathologiques et, en particulier, des inflammations et des tumeurs. Ces anticorps anti-nitrotyrosine sont également utiles comme moyen de ciblage d'agent thérapeutique dans des sites tissulaires sujets à des dommages résultant de l'oxydation. Ces anticorps anti-nitrotyrosine ne sont pas des moyens de détection spécifiques de pathologies ostéo-articulaires et permettent seulement de mettre en évidence, voire de traiter, sans discernement les cancers, les maladies d'Alzheimer, la maladie de Parkinson, les maladies inflammatoires intestinales, le lupus érythémateux, l'arthrose ou la polyarthrite rhumatoïde. Ces anticorps anti-nitrotyrosine ciblent indifféremment toutes les protéines comportant des acides aminés de type nitrotyrosine, de sorte qu'il n'est pas possible d'identifier le tissu ou la protéine détectée. De la même façon, la demande PCT WO-A-98/29452 divulgue un anticorps reconnaissant spécifiquement une protéine nitrosylée, en particulier l'albumine nitrosylée.

Dans ces deux demandes PCT et dans l'article de Ze Y Z, le marqueur biologique ou biochimique est la nitrotyrosine indépendamment de la séquence d'acides aminés avoisinante.

La demande PCT WO-A-00/79284 décrit une série de marqueurs spécifiques de la dégradation du collagène comprenant la séquence QYDSYD ou la séquence JYDSYD avec J étant l'acide pyroglutamique, ainsi que des anticorps dirigés contre ces marqueurs.

La demande de brevet PCT WO-A-03/076946 divulgue un marqueur biologique ou biochimique constitué, par exemple, par une séquence peptidique du type HRGYPGLDG [SEQ ID N°13] dans laquelle les résidus tyrosine Y sont nitrosylés ou nitratés, ou par une séquence LQYMRA [SEQ ID N°14]. Ces deux séquences peptidiques porteuses de nitrotyrosine sont spécifiques de collagène de type II, qui est le composant majoritaire de la matrice extracellulaire du cartilage synthétisée par les chondrocytes.

Cependant, la dégradation du tissu cartilagineux n'est pas le signe le plus précoce des pathologies articulaires.

Il existe pourtant un besoin important pour des applications diagnostic, suivi, pronostic et thérapeutique, d'un marqueur précoce ou pré-symptomatique de pathologie articulaire, tant il est difficile, comme cela a été souligné ci-avant, de diagnostiquer rapidement de telles maladies, la douleur, la raideur de l'articulation et la radiographie, utilisées à ce jour étant des signes tardifs de telles maladies articulaires.

Et à ce jour, à la connaissance de la demanderesse, le seul marqueur spécifique de tissu synovial indicateur précoce pré-symptomatique de pathologie ostéo-articulaire, est la pyridinoline glycosylée et plus particulièrement la pyridinoline diglycosylée (Pyr-Gal-Glc), appelée glusosyl-galactosyl-pyridinoline ou pyridinoline disaccharidique. Ce marqueur précoce spécifique de pathologie synoviale ainsi que les méthodes et les kits faisant application dudit marqueur sont décrits dans la demande de brevet français FR-A-2 795 823. La pyridinoline disaccharidique (Pyr-Gal-Glc) est spécifique du collagène synovial, c'est-à-dire d'un collagène de type I, III, IV, V ou VI ou d'un mélange de ceux-ci, de préférence majoritairement de collagènes de types I et III.

Les documents US-A-2003/119058, US-B-6 355 442, US-B-6 342 361, US-B-6 323 314, US-B-6 110 689 divulguent des méthodes de dosage de produits de dégradation de collagène du type III, à des fins de diagnostic de désordres liés au métabolisme du collagène et, plus particulièrement, à la maladie osseuse dénommée ostéoporose. Les collagènes concernés sont notamment les collagènes de types I, II et III. Parmi les séquences peptidiques utilisées comme marqueurs et divulguées dans ces documents, figure la séquence peptidique localisée à l'extrémité N-terminale d'un télopeptide d'un collagène de type III : DVKSGV [SEQ ID N°12] La pathologie osseuse visée dans ces brevets, à savoir l'ostéoporose, diffère clairement des pathologies articulaires inflammatoires assorties de dégradations oxydatives. La SEQ ID N°12 ne comprend pas de tyrosine nitrosylée.

Le document WO 00/79284 divulgue des moyens de détection (anticorps et séquence peptidique) de produits de dégradation de collagène du type II et III, à des fins de diagnostic d'une éventuelle résorption du cartilagineux non-minéralisé in vivo.

Dans le même registre, l'EP-B-0 362 235 décrit un procédé d'analyse de produits de décomposition du collagène de type III, procédé dans lequel on fait intervenir un anticorps spécifique du télopeptide aminoterminal du collagène de type III. Cet anticorps est apte à reconnaître et à se lier avec la séquence peptidique constitutive de tout le télopeptide aminoterminal. La détection du télopeptide aminoterminal de collagène de type III selon l'EP-B-0 362 235 n'est pas reliée à la mise en évidence de pathologies ostéo-articulaires inflammatoires oxydatives.

### PROBLEME A RESOUDRE - OBJECTIFS

Sur un plan général, l'un des objectifs majeurs de l'invention est de fournir des outils et des méthodes permettant d'améliorer le diagnostic, le traitement, le suivi et le pronostic de maladies qui s'accompagnent d'une Dégradation Oxydative de Tissus contenant du Collagène de type III (lesdites maladies étant ci-après qualifiées de D.O.T.Coll.III), en particulier les affections inflammatoires telles que les maladies ostéo-articulaires.

Sur un plan plus spécifique et compte tenu de l'intérêt médical dont est porteur un marqueur spécifique et précoce ou pré-symptomatique de pathologies ostéo-articulaires, l'un des objectifs essentiels de la présente invention est de fournir un nouveau marqueur de ce type susceptible d'être une alternative à la pyridinoline disaccharique (Pyr-Gal-Glc) divulguée à cette fin dans la demande de brevet FR-A-2 795 823.

On rappellera en effet que dans certaines pathologies ostéo-articulaires (en particulier la polyarthrite rhumatoïde), les tissus non minéralisés (synoviale, cartilage) sont dégradés avant la matrice osseuse.

Pour améliorer encore la spécificité des marqueurs spécifiques et précoces ou pré-symptomatiques de pathologies ostéo-articulaires (notamment synoviale), il serait intéressant de disposer d'un marqueur encore plus spécifique de ce type d'affections articulaires inflammatoires. C'est un autre objectif essentiel de la présente invention.

Un autre objectif essentiel de l'invention est de fournir un marqueur spécifique et précoce ou pré symptomatique du métabolisme oxydatif du tissu synovial et plus particulièrement de la nitrolysation du collagène synovial.

Un autre objectif essentiel de l'invention est de fournir un marqueur spécifique et précoce ou pré-symptomatique du métabolisme oxydatif de tissus contenant du collagène de type III, tel que le tissu synovial et plus particulièrement de la nitrosylation d'un collagène de type III.

Un autre objectif essentiel de la présente invention est de fournir un nouveau marqueur peptidique, d'une part, simple, sensible et fiable et, d'autre part, spécifique des pathologies D.O.T.Coll.III, dont les pathologies ostéo-articulaires, plus précisément du métabolisme oxydatif des tissus contenant du collagène, tel que le tissu synovial et, encore plus précisément, de la nitrolysation du collagène III e.g. synovial, en vue d'améliorer la précocité des informations cliniques susceptibles d'être obtenues pour des pathologies D.O.T.Coll.III, notamment ostéo-articulaires, de façon à permettre in fine, un meilleur diagnostic, un meilleur suivi, un meilleur pronostic et un meilleur traitement desdites pathologies.

Un autre objectif de l'invention est d'améliorer les méthodes de diagnostic des maladies D.O.T.Coll.III, notamment ostéo-articulaires, en particulier des maladies synoviales.

Un autre objectif essentiel de l'invention est de fournir de nouvelles méthodes de diagnostic économiques, performantes, fiables et réalisables simplement in vitro à partir d'un échantillon biologique susceptible de contenir des résidus protéiques dégradés, en particulier des résidus collagéniques, topiques des affections D.O.T.Coll.III, notamment ostéo-articulaires.

Un autre objectif essentiel de l'invention est de fournir les moyens utiles pour ces méthodes de diagnostic, en particulier les marqueurs spécifiques (séquences peptidiques, de préférence collagéniques) et les éléments de détection (anticorps) desdits marqueurs.

Un autre objectif essentiel de l'invention est de fournir des méthodes améliorées pour le suivi, le pronostic de pathologies D.O.T.Coll.III, notamment ostéo-articulaires [ostéo-articulaires, en particulier synoviales] qui soient précoces fiables, performantes, économiques, et qui soient réalisables simplement in vitro, à partir d'un échantillon biologique d'un individu susceptible de contenir des résidus protéiques, en particulier collagéniques, de dégradation rendant compte de l'état pathologique.

Un autre objectif essentiel de l'invention est de fournir des méthodes améliorées pour la détermination de l'efficacité ou la toxicité d'un médicament adapté au traitement d'une pathologie D.O.T.Coll.III, notamment ostéo-articulaire (e.g. synovial).

Un autre objectif essentiel de la présente invention est de fournir des kits pour la mise en oeuvre des méthodes visées dans les objectifs ci-dessus.

Un autre objectif essentiel de l'invention est de fournir des éléments de détection, en particulier des anticorps spécifiques du marqueur susvisé et susceptible d'être contenu dans les moyens de diagnostic de suivi, de pronostic ou de détermination de la toxicité ou de l'efficacité d'un remède contre ces pathologies D.O.T.Coll.III, notamment synoviales ou ostéo-articulaires.

Un autre objectif essentiel de l'invention est de fournir des séquences peptidiques spécifiques des maladies où interviennent des modifications post-traductionnelles, dégradation de protéines, par exemple de collagène, en particulier de collagène de type III sous l'effet d'une espèce hyper-oxydante, notamment du monoxyde d'azote NO et de ses dérivés (maladies D.O.T.Coll.III telles que les arthrites, cancers, maladies neuro-dégénératives).

Un autre objectif essentiel de l'invention est de proposer l'utilisation de ces séquences peptidiques pour des déterminations rapides et précoces in vitro de l'existence de pathologies donnant lieu à un stress oxydatif du tissu synovial.

Un autre objectif essentiel de l'invention est de fournir des séquences nucléiques pour les marqueurs peptidiques susvisés de dégradation oxydative.

### BREVE DESCRIPTION DE L'INVENTION

Pour atteindre ces objectifs, parmi d'autres, les inventeurs ont eu le mérite de mettre en évidence, un nouveau marqueur peptidique (IIINys) localisé au niveau du télopeptide N-terminal du collagène du type III. Ce nouveau marqueur est spécifique du métabolisme oxydatif de tissus contenant du collagène de type III tels que le tissu synovial et plus particulièrement de la nitrolysation du collagène III, en particulier synovial.

Sachant que les tissus contenant du collagène de type III tels que le tissu synovial est le premier à être dégradé dans les pathologies D.O.T.Coll.III notamment les maladies ostéo-articulaires, ce nouveau marqueur est un marqueur précoce ou encore un marqueur pré-symptomatique desdites pathologies.

Ce nouveau marqueur est d'autant plus intéressant qu'il peut être détecté qualitativement et quantitativement dans des échantillons biologiques (sang, urine) in vitro, provenant d'individus atteints de pathologies qui s'accompagnent de dégradation oxydative du type nitrolysation de télopeptides amino-terminaux de collagène de type III, telles que les maladies ostéo-articulaires, en particulier synoviales. Cette détection in vitro peut être réalisée de façon simple, rapide et économique, à l'aide d'éléments de détection du type anticorps, en mettant en oeuvre des moyens de révélation du complexe anticorps/antigène, porteurs d'au moins un épitope constitué par le marqueur.

Le nouveau marqueur selon l'invention peut donc être avantageusement associé à des kits de détection, à des moyens de détection de type anticorps et à des moyens de production desdits anticorps (cellules -hybridomes- séquences nucléiques).

D'où il s'ensuit que dans le premier de ses aspects, la présente invention concerne un nouveau marqueur spécifique d'une dégradation oxydative de tissus contenant du collagène de type III, de préférence de la nitrosylation du collagène de type III, caractérisé en ce qu'il comprend au moins une séquence peptidique SP* de 6 à 25 acides aminés (AA), de préférence de 6 à 20 AA, et, plus préférentiellement encore, de 6 à 15 AA, ladite séquence peptidique SP* comprenant la sous-séquence QYDSYD [SEQ ID N°1] dans laquelle au moins l'un des Y est nitrosylé* et où Q correspond non seulement à la glutamine conformément au code international mais aussi à l'acide pyroglutamique.

La présence, voire la concentration d'un tel marqueur dans un échantillon biologique d'un individu, donne des informations précoces et fiables sur des dommages oxydatifs dans un tissu de cet individu, dommages le plus souvent liés à une inflammation. En d'autres termes, cette séquence peptidique SP* marqueuse sous forme nitrosylée, constitue un index de l'état pathologique d'un individu atteint ou susceptible d'être atteint par des maladies D.O.T.Coll.III, notamment inflammatoires, telles que les maladies ostéo-articulaires, le cancer, les maladies neurodégénératrices (Parkinson, Alzheimer).

De préférence, dans le cas où les tissus contenant du collagène de type III et ayant subis ou subissant des dégradations oxydatives, sont des tissus synoviaux, le collagène ciblé par le marqueur renvoie à des pathologies synoviales, c'est-à-dire des pathologies correspondant à une augmentation du turnover, à une prolifération, une dégradation, une inflammation, une destruction, une décomposition, un remodelage pathologique ou une dégradation de la synoviale ou du collagène synovial.

Le collagène synovial signifie, par exemple, au sens du présent exposé, un collagène de types, I, III, IV, V ou VI, ou un mélange de ces collagènes. De préférence, il s'agit d'un collagène de type III.

Le marqueur selon l'invention est un marqueur spécifique de pathologie synoviale permettant de distinguer cette dernière parmi d'autres pathologies et notamment les pathologies atteignant l'os et/ou le cartilage.

Outre les pathologies synoviales, l'invention concerne également d'autres pathologies ostéo-articulaires, dont celles qui entraînent une prolifération de la synoviale et une atteinte du cartilage. Ces pathologies ostéo-articulaires peuvent être des rhumatismes inflammatoires, des arthropathies métaboliques, les rhumatismes dégénératifs, des polyarthrites rhumatoïdes, des spondylarthropathies, la goutte, la chondrocalcinose ou l'arthrose, entre autres.

### DESCRIPTION DETAILLE DE L'INVENTION

Suivant une caractéristique préférée de l'invention, la séquence peptidique SP* du marqueur est choisie dans le groupe de séquences suivantes:
- QY*DSY*DVKSG [SEQ ID N°2];
- QY*DSYDVKSG [SEQ ID N°3];
- QYDSY*DVKSG [SEQ ID N°4];
séquences dans lesquelles Y* correspond à un Y nitrosylé.

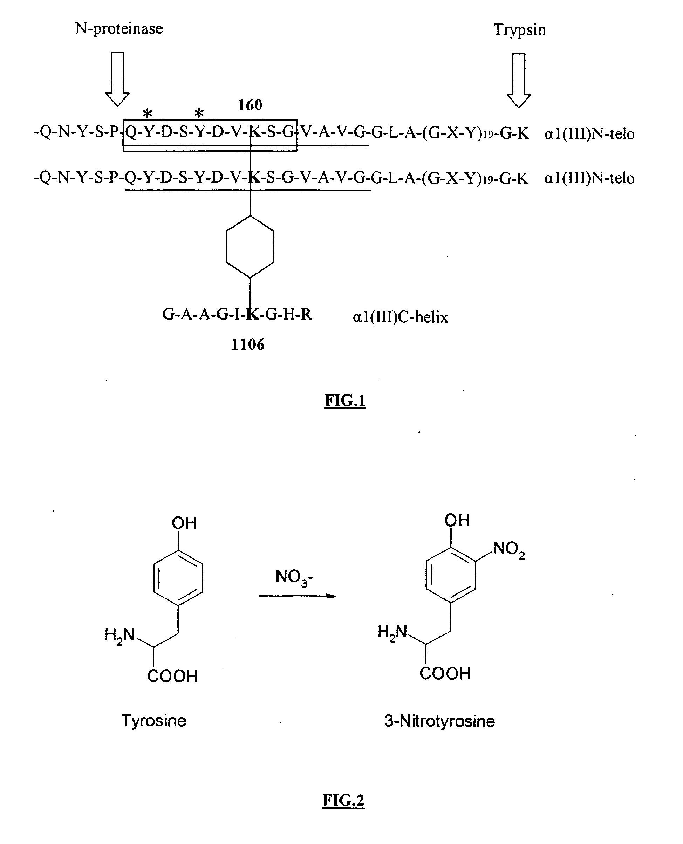

Au sens de l'invention, le terme "nitrosylé" correspond, par exemple, à la modification post-traductionnelle que subit la tyrosine pour devenir la 3-nitrotyrosine. Cet exemple de nytrosylation est illustré par la figure 2 annexée.

Selon une variante, la séquence peptidique SP* est choisie dans le groupe de séquences suivantes:
- QY*DSY*DVKS [SEQ ID N°5];
- QY*DSYDVKS [SEQ ID N°6];
- QYDSY*DVKS [SEQ ID N°7];
séquences dans lesquelles Y* correspond à un Y nitrosylé.

Le marqueur selon l'invention peut comprendre plusieurs séquences peptidiques SP* de nature différente, par exemple un mélange constitué d'au moins deux des séquences SEQ ID N°2 à SEQ ID N°7.

Il est également envisageable, conformément à l'invention, que le marqueur comprenne en sus de la ou des séquences peptidiques SP*, la séquence peptidique SP non nitrosylée : QYDSYDVKSG [SEQ ID N°8].

Il est à noter que les séquences peptidiques SP*, en particulier SEQ ID N°2, 3, 4, 5, 6 et 7 comportent un reste de lysine apte à former un pontage avec d'autres restes de lysine appartenant à une chaîne peptidique de la même molécule protéique ou à une autre molécule protéique.

En outre, le pontage peut impliquer deux protéines, par exemple collagéniques, identiques ou différentes.

Dans le cas où ces deux protéines sont identiques, la séquence peptidique SP* ou SP, contenue dans une protéine P est reliée par au moins un pontage à au moins une autre séquence peptidique SP* ou SP, contenue dans la même molécule de protéine P ou à une autre séquence peptidique SP', contenue dans une autre molécule de protéine P, ledit pontage reliant de préférence un reste de lysine d'une séquence à un reste de lysine de l'autre séquence, et, plus préférentiellement encore, ledit pontage comprenant une liaison covalente directe ou au moins un reste pyridinoline.

Dans le cas où ces deux protéines sont différentes, la séquence peptidique SP* ou SP, contenue dans une protéine P est reliée par au moins un pontage à au moins une autre séquence peptidique SP^{x}, contenue dans une protéine P^{x} différente de P, ledit pontage reliant de préférence un reste de lysine d'une séquence à un reste de lysine de l'autre séquence, et, plus préférentiellement encore, ledit pontage comprenant une liaison covalente directe ou au moins un reste pyridinoline.

Dans un mode préféré de réalisation de l'invention, la séquence peptidique SP*, voire la séquence peptidique SP est (sont) contenu(s) dans une protéine P constituée par du collagène de type III, dans un télopeptique (avantageusement aminoterminal) dudit collagène de type III.

En revanche, comme indiqué ci-dessus, la protéine P^{x}, éventuellement reliée par un pontage à la protéine P porteuse de la séquence SP* et éventuellement SP, est de préférence un collagène de type différent du type III, et, plus préférentiellement encore, un collagène de type I, SP^{x} étant de préférence contenu dans un télopeptide (avantageusement aminoterminal).

La séquence peptidique SP' différente de SP* et de SP, peut être localisée n'importe où dans la protéine P, c'est-à-dire dans le type triple hélice ou dans les télopeptides lorsqu'il s'agit de collagène. La localisation de SP peut, par exemple, être dans la partie C-terminale de la triple hélice de collagène de type III.

Par "protéine", on entend au sens de l'invention, la protéine entière, par exemple le collagène ou des fragments de celle-ci résultant de la dégradation oxydative liée à une pathologie D.O.T.Coll.III, par exemple une pathologie inflammatoire articulaire.

Selon un deuxième de ses aspects, la présente invention concerne une séquence peptidique isolée du corps humain ou autrement produite par un procédé technique, caractérisée en ce qu'elle comprend de 6 à 25 acides aminés (AA), de préférence de 6 à 20 AA, et, plus préférentiellement encore, de 6 à 15 AA, ladite séquence peptidique comprenant la sous-séquence QYDSYD [SEQ ID N°1] dans laquelle au moins l'un des Y est nitrosylé* et où le Q peut être l'acide pyroglutamique.

De préférence, cette séquence peptidique est caractérisée en ce qu'elle est choisie dans le groupe de séquences suivantes:
- SP* telle que définie ci-dessus,

Dans ce deuxième aspect de l'invention, on vise les séquences peptidiques prises en tant que telles, à titre de produits nouveaux, et utilisables notamment comme marqueur spécifique d'une dégradation oxydative de tissus contenant du collagène III, par exemple le tissu synovial.

Suivant un troisième aspect, l'invention vise les outils génétiques de synthèse des séquences peptidiques visées ci-dessus, et en particulier les séquences SP* ou SP, plus particulièrement encore les séquences N°2, 3, 4, 5, 6 et 7. Dans ce troisième aspect, l'invention vise donc toute séquence nucléique codant pour le marqueur défini ou la séquence définie ci-dessus.

Dans le quatrième de ses aspects, la présente invention concerne l'utilisation d'au moins une séquence peptidique SP* ou SP telles que définies ci-dessus dans la présentation du marqueur selon le premier aspect de l'invention, ou d'une séquence peptidique prise en tant que telle conformément au troisième aspect de l'invention (par exemple SEQ ID N°1, 2, 3, 4, 5, 6 et 7), pour une application technique choisie dans le groupe comprenant:
- le diagnostic d'une pathologie qui s'accompagne d'une dégradation oxydative de tissus contenant du collagène de type III;
- le suivi d'une pathologie qui s'accompagne d'une dégradation oxydative de tissus contenant du collagène de type III;
- la détermination de l'efficacité d'un médicament adapté au traitement d'une pathologie qui s'accompagne d'une dégradation oxydative de tissus contenant du collagène de type III;
- ou la détermination de la toxicité d'un médicament adapté au traitement d'une pathologie qui s'accompagne d'une dégradation oxydative de tissus contenant du collagène de type III.

De préférence, la pathologie qui s'accompagne d'une dégradation oxydative de tissus contenant du collagène de type III (pathologie D.O.T.Coll.III), est une pathologie synoviale ou une autre pathologie ostéo-articulaire.

L'utilisation selon le quatrième aspect de l'invention est à relier directement au cinquième aspect de la présente invention, à savoir :
→ d'une part, une méthode de diagnostic, de suivi ou de pronostic d'une pathologie qui s'accompagne d'une dégradation oxydative de tissus contenant du collagène de type III , caractérisée en ce qu'elle comprend les étapes essentielles suivantes:
   a. analyse quantitative et/ou qualitative in vitro d'un échantillon biologique d'un individu, afin de détecter un marqueur spécifique d'une dégradation oxydative du tissu synovial selon l'une quelconque des revendications 1 à 8;
   b. comparaison de la concentration en marqueur éventuellement détecté dans l'échantillon avec une concentration de référence correspondant à l'existence, à un stade déterminé, ou à l'absence chez l'individu, d'une pathologie synoviale ou d'une autre pathologie ostéo-articulaire ;
→ et, d'autre part, une méthode pour la détermination de l'efficacité ou de la toxicité d'un médicament adapté au traitement d'une pathologie qui s'accompagne d'une dégradation oxydative de tissus contenant du collagène de type III, caractérisée en ce qu'elle comprend les étapes essentielles suivantes :
   a. analyse quantitative et/ou qualitative in vitro d'un échantillon biologique d'un individu, afin de détecter un marqueur spécifique d'une dégradation oxydative du tissu synovial selon l'une quelconque des revendications 1 à 8;
   b. comparaison de la concentration en marqueur éventuellement détecté dans l'échantillon avec une concentration de référence correspondant à l'existence, à un stade déterminé, ou à l'absence chez l'individu, d'une pathologie synoviale ou d'une autre pathologie ostéo-articulaire.

De préférence, la pathologie qui s'accompagne d'une dégradation oxydative de tissus contenant du collagène de type III (pathologie D.O.T.Coll.III), est une pathologie synoviale ou une autre pathologie ostéo-articulaire.

Les méthodes selon l'invention sont des méthodes médicales de prévention et de traitement de pathologies qui s'accompagnent d'une dégradation oxydative de tissus contenant du collagène de type III (pathologie D.O.T.Coll.III), de préférence des pathologies synoviales ou des pathologies ostéo-articulaires.

Elles consistent à détecter dans des échantillons biologiques, le marqueur spécifique selon l'invention tel que défini ci-avant. Un échantillon biologique peut comprendre notamment un fluide biologique, en particulier ceux qui sont habituellement testés en tant qu'échantillon clinique. Cela inclut tous les liquides corporels tels que le sang (sous toutes les formes analysables, en particulier le sérum et le plasma), l'urine, la salive, la sueur, les extraits ou les surnageants de culture ou de cellule ou le liquide synovial. Le liquide synovial est avantageux pour la détection de pathologies synoviales dans la mesure où il est spécifique du tissu synovial ou de la synoviale. Un échantillon biologique peut être également un tissu extrait de l'individu, ce tissu pouvant éventuellement être mis en culture avant que l'on effectue la détection du marqueur sur ce tissu. Ce tissu peut être par exemple la synoviale.

Dans les méthodes selon l'invention, la détection du marqueur dans l'échantillon biologique est qualitative et/ou quantitative. Cette détection peut être effectuée par exemple par une technique de chromatographie liquide à haute performance (HPLC), par fluorescence, par spectroscopie aux ultraviolets, par détection électrochimique ou par les techniques d'analyse protéomique ou "microarray".

De préférence, la détection sera plutôt fondée sur une technique immunologique (e.g. ELISA, technique immuno-enzymatique, technique d'immuno-fluorescence, technique radio-immunologique, technique chémo-immunologique).

Ces différentes techniques de détection sont bien connues de l'homme de l'art. S'agissant des techniques immunologiques, on pourra se référer à Diamantis et Cristopoulos, Immunoassay, Academic Press, San Diego 1996, notamment les pages 579 et suivantes. Pour les techniques d'analyse protéomique, on pourra notamment se référer à Urbanowska et al. Development of protein microaray technology to monitor biomarkers of rheumathoid arthritis disease. Cell Biol. Toxicol., 2003 19(3): 189-202 à Glokler and Angenendt. Protein and antibody microarray technology. Journal of chromatography B, 2003 797(1-2): 229-240.

Concernant la "concentration de référence" qui sert de base de comparaison dans les méthodes selon l'invention, les précisions suivantes peuvent être apportées.

Le choix de la concentration de référence correspondant soit à une absence de pathologie, soit à l'existence d'une pathologie à un stade déterminé, peut être fait par l'homme du métier en fonction du marqueur sélectionné ainsi que de la technique utilisée.

A titre d'exemple, la concentration de référence qui délimite l'état pathologique de l'état non pathologique pour différentes techniques, correspond à la moyenne des malades au dessus du 95^{ième} percentile des valeurs des contrôles.

Puisque l'invention concerne dans son cinquième aspect, des méthodes faisant intervenir la détection du marqueur, il est clair qu'un sixième aspect de l'invention porte sur les moyens permettant la mise en oeuvre de ces méthodes.

Ainsi, l'invention concerne un moyen pour le diagnostic, le suivi ou le pronostic d'une pathologie qui s'accompagne d'une dégradation oxydative de tissus contenant du collagène de type III, caractérisé en ce qu'il comprend au moins un élément de détection du marqueur tel que défini ci-dessus.

Un autre moyen selon l'invention est un moyen pour la détermination de l'efficacité d'un médicament adapté au traitement d'une pathologie qui s'accompagne d'une dégradation oxydative de tissus contenant du collagène de type III, caractérisé en ce qu'il comprend au moins un élément de détection du marqueur tel que défini ci-dessus.

Un autre moyen selon l'invention est un moyen pour la détermination de la toxicité d'un médicament adapté au traitement d'une pathologie qui s'accompagne d'une dégradation oxydative de tissus contenant du collagène de type III, caractérisé en ce qu'il comprend au moins un élément de détection du marqueur tel que défini ci-dessus.

Comme indiqué ci-dessus dans le cadre de l'évocation des méthodes, la détection suivant l'invention est une détection de type immunologique. Ainsi, conformément à l'invention, les moyens susvisés ont pour caractéristique que l'élément de détection tel que défini ci-dessus est caractérisé en ce que l'élément de détection comporte au moins un anticorps (purifié) apte à reconnaître l'épitope compris dans la séquence peptidique SP* du marqueur tel que défini ci-dessus et à s'associer de manière spécifique avec la protéine porteuse dudit marqueur ou de ladite séquence.

Les moyens de l'invention s'appliquent pour des pathologies qui s'accompagnent d'une dégradation oxydative de tissus contenant du collagène de type III (pathologie D.O.T.Coll.III), et, de préférence, pour des pathologies synoviales ou d'autres pathologies ostéo-articulaires.

Dans un septième aspect, l'invention concerne, à titre de produit nouveau *per se,* un anticorps (purifié) apte à reconnaître l'épitope compris dans la séquence peptidique SP* du marqueur tel que défini ci-dessus ou la séquence telle que définie ci-dessus et à s'associer de manière spécifique avec la protéine porteuse dudit marqueur ou de ladite séquence.

Par "anticorps" au sens de la présente invention, on entend, par exemple, les anticorps monoclonaux et polyclonaux ou tout autre fragment dérivé des anticorps, les anticorps humanisés, les fragments Fab, les anticorps chimères ou tout autre fragment de complexe antigène-anticorps spécifique, entre autres. Quand l'anticorps est utilisé dans le contexte de la méthode de détection de fragment de collagène (protéine P), l'expression "anticorps" comprend tout analyte produit spécifique du fragment de collagène contenant au moins la SEQ ID N°1.

Par "épitope", on désigne par exemple une séquence minimum d'acides aminés reconnus par l'anticorps dans la protéine P porteuse et/ou dans les séquences peptidiques SP*, voire SP. L'épitope détermine l'immunospécificité. Dans le contexte de l'invention, un épitope peut être une courte séquence de trois acides aminés contigus ou une séquence plus longue contenant au moins quatre acides aminés.

Dès lors que la protéine P au sens de la présente invention peut contenir des fragments protéiques, ceux-ci peuvent être des polypeptides, c'est-à-dire des molécules peptidiques contenant plus de 50 acides aminés.

Suivant une caractéristique préférée de l'invention, les anticorps utiles comme élément de détection dans les méthodes ou les moyens considérés, peuvent être monoclonaux ou polyclonaux. La production de ces anticorps se fait selon les méthodes standards bien connues de l'homme de l'art et décrites notamment par HARLOW et LANE en 1988, ainsi que par KÖHLER et MILSTEIN en 1975 dans "Continuous cultures of fused cells secreting antibody of predefine specificity" Nature 1975, 256(5517):495-7. En particulier, on préparera par exemple selon les synthèses peptidiques classiques, les marqueurs à détecter que l'on couplera à une protéine dite "carrier" qui est préférentiellement la KLH (Keyhole limpet hemocyanine) ou l'albumine bovine ou humaine sérique, l'ovalbumine, la tyroglobuline ou toute autre protéine "carrier" comme l'edestine, la toxine tétanique ou cholérique, les polyaminoacides comme la poly-D-Lysine-D-acide glutamique. Ce couplage sera réalisé via une cystéine, une glutaraldéhyde, ou un carbodiimide par exemple. Cette séquence couplée permet alors l'immunisation de souris, rat, lapin, poulet, ou tout autre individu permettant la production d'anticorps.

Les anticorps selon l'invention sont spécifiques car ils reconnaissent les fragments de dégradation du collagène de type III contenant au moins quatre aminoacides consécutifs SEQ ID N°1, de préférence au moins cinq, présents dans le télopeptideaminoterminal du collagène de type III.

L'obtention d'un anticorps spécifique des marqueurs peptidiques SP*, voire SP selon l'invention, peut nécessiter que l'antigène soit, dans une première étape, extrait et purifié à partir d'urine, par exemple. Une telle extraction ou purification peut être réalisée de manière avantageuse par la technique décrite dans l'exemple et également par la synthèse de peptides synthétiques.

Il est à noter que, selon une particularité avantageuse de l'invention, les moyens utiles dans les méthodes selon l'invention, en particulier les éléments de détection que sont par exemple les anticorps spécifiques des marqueurs, ont pour propriété de détecter les pontages entre au moins deux séquences peptidiques SP ou entre au moins une séquence peptidique SP* et au moins une séquence peptidique SP, ou bien encore au moins une séquence peptidique SP* et au moins une autre protéine P^{x}. Ces pontages s'effectuent de préférence entre des restes lysyls des différentes séquences peptidiques en cause.

Pour le moins, la protéine P est de préférence le collagène de type III et la protéine P^{x} est de préférence un collagène de type I.

Dans son huitième aspect, l'invention vise d'une part un kit de diagnostic de suivi ou de pronostic d'une pathologie qui s'accompagne d'une dégradation oxydative de tissus contenant du collagène de type III, et d'autre part, un kit pour la détermination de l'efficacité ou de la toxicité d'un médicament adapté au traitement d'une pathologie qui s'accompagne d'une dégradation oxydative de tissus contenant du collagène de type III. Ces deux types de kit ont pour caractéristique de comprendre au moins un moyen tel que défini ci-dessus.

Ces kits comprennent des éléments de détection ou réactif (par exemple anticorps anti-marqueur tels que définis ci-dessus) destinés à être mis en contact avec l'échantillon biologique à analyser. Ces kits comportent également des moyens de révélation de la formation des complexes antigènes/anticorps (par exemple anticorps secondaires, indicateurs colorés, indicateurs fluorescents, indicateurs radioactifs, etc. Classiquement, est également prévu dans ces kits, un protocole opératoire permettant de réaliser l'analyse, la lecture des résultats révélés par les moyens révélateurs s'opère à l'oeil nu pour une analyse qualitative et à l'aide d'instruments de mesure plus élaborés pour les dosages quantitatifs. De préférence, ces kits visent des pathologies synoviales ou d'autres pathologies ostéo-articulaires.

L'invention sera mieux comprise et ses avantages ressortiront des exemples qui suivent, qui décrivent la préparation d'un exemple de marqueur selon l'invention, la préparation d'éléments de détection constitués par des anticorps polyclonaux spécifiques dudit marqueur et une méthode de détection par une technique immunologique de type ELISA de compétition du marqueur selon l'invention, et enfin la mise en évidence de la détection de malades atteints de polyarthrite rhumatoïde sur des échantillons sériques provenant de ces malades au moyen du marqueur et de la méthode selon l'invention. Les figures 1, 2, 3, 4, 5, 6A ET 6B annexées complètent l'information donnée par les exemples qui suivent.

### DESCRIPTION DES FIGURES 1, 2, 3, 4, 5, 6A et 6B

- La figure 1 représente la structure du télopeptide aminoterminal du collagène de type III pontée avec deux autres molécules de collagène de type III par la pyridinoline ou un des ses dérivés. La séquence encadrée est la SEQ ID N°2 qui correspond à un mode préféré de réalisation du marqueur selon l'invention, ci-après dénommé IIINys. Les astérisques indiquent les deux résidus de tyrosine qui sont nitrosylés
- La figure 2 représente la formation de la 3-nitrotyrosine par action du peroxynitrite sur la tyrosine.
- La figure 3 représente la courbe standard type de l'ELISA dosant le peptide IIINys de séquence SEQ ID N°2
   La figure 4 illustre la spécificité des anticorps polyclonaux préparés vis-à-vis du IIINys, par rapport à d'autres peptides synthétiques. B/B0 représente le ratio entre la quantité d'anticorps anti-IIInys fixé sur l'antigène fixé ("coaté") sur la plaque en présence du composé compétiteur (B) et en l'absence du composé compétiteur (B0).
   -•- QY*DSY*DVKSG SEQ ID N°2
   -▲- QYDSYDVKSG SEQ ID N°8
   -■- 3-nitro tyrosine SEQ ID N°12
   -×- nitrated BSA SEQ ID N°11
   -◆- PQY*DSY*DVKSG SEQ ID N°9
   -□- QY*DSYDVKSG SEQ ID N°3
   -○- QYDSY*DVKSG SEQ ID N°4
- La figure 5 montre la concentration sérique C en IIINys (µg/L) chez des adultes sains (n=29) [CTRL] et des patients atteints de polyarthrite rhumatoïde (n=30) [RA].
- La figure 6A représente une analyse immunohistochimique de sections de tissus synoviaux de patients atteints d'arthrose du genou marquées avec le sérum pré-immun du lapin ayant produit l'antisérum IIINys (sérum prélevé avant l'immunisation et servant de contrôle négatif).
- La figure 6B représente montre une analyse immunohistochimique de sections de tissus synoviaux de patients atteints d'arthrose du genou marquées avec l'anticorps IIINys. Les flèches notées sur la figure montrent le marquage par l'anticorps IIINys.

### EXEMPLES

A titre d'illustration non limitative de l'invention, la figure 1 représente les séquences peptidiques de deux télopeptides aminoterminaux d'un collagène de type III, dont l'un comporte une séquence peptidique SP* SEQ ID N° 2 constituant un mode préféré de réalisation du marqueur IIINys selon l'invention, ces deux télopeptides étant en outre reliés par pontage avec la partie en triple hélice d'une autre molécule de collagène de type III. Le domaine télopeptidique non hélicoïdal est souligné. Les flèches représentent les sites de clivage de la trypsine et de la N-protéinase. Les astérisques surmontant les codes Y indiquent les résidus tyrosine ou les restes tyrosine nitrosylés. Le pontage est un pontage de type pyridinoline reliant les deux télopeptides d'une molécule de collagène de type III avec la région C-terminale de l'hélice d'une autre molécule de collagène de type III.

### A - METHODES

### 1. Préparation du peptide synthétique

La préparation des peptides synthétiques peut être réalisée selon les techniques largement décrites dans la littérature scientifique. Le peptide IIINys de séquence SEQ ID N° 2 dérivé de la séquence du télopeptide N-terminal du collagène de type III (numéro d'accession dans GenBank : P02461 : SEQ ID N°10) a été produit par la technique de synthèse peptidique sur phase solide en utilisant un blocage réversible de l'amine de l'acide aminé par le Fmoc (9-Fluorenylmethyloxycarbonyl). La pureté du peptide a été vérifiée par HPLC et par spectrométrie de masse.

Le peptide servant d'immunogène, synthétisé avec une cystéine supplémentaire du côté C-terminal, a été couplé du côté C-terminal à une protéine porteuse qui est la KLH (keyhole limpet hemocyanine) ce qui va permettre une meilleure réponse immunologique vis-à-vis de ce peptide. Le peptide synthétique qui est adsorbé sur les microplaques a été couplé à la biotine (Harlow et Lane, 1988).

### 2. Préparation des anticorps polyclonaux

Les lapins ont subi une injection par voie intra-péritonéale du peptide conjugué à la KLH émulsifié avec de l'adjuvant complet de Freud. Cette opération a été répétée 3 fois pendant trois mois en utilisant le même peptide avec de l'adjuvant incomplet de Freud. Après chaque injection un échantillon de sang de lapin a été prélevé. On a laissé coaguler ce sang pendant 30 min et le tout a été centrifugé pendant 10 min à 2500 rpm pendant 10 min afin de récupérer le sérum. Dix jours après la dernière injection, les lapins ont été sacrifiés et tout le sang a été récupéré. Chaque prélèvement sérique a été titré par technique ELISA en présence du peptide synthétique. L'antisérum avec le meilleur titre a été sélectionné pour la mise au point de l'ELISA de compétition (Harlow et Lane, 1988a).

### 3. Titration des antisérums

Les titres des antisérums ont été réalisés en adsorbant le peptide conjugué à la biotine dilué avec du tampon de revêtement ("coating") sur des plaques streptavidine (Nunc, Danemark) 2h à température ambiante. Après lavage, 50 µl d'antisérums dilué du 1/100 au 1/1.000.000 sont ajoutés à 50 µl de tampon de dilutions dans les puits et sont mis à incuber pendant une nuit à 4°C. Après lavage, 100 µl d'anticorps secondaire couplés à la peroxydase dirigé contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

### 4. ELISA de compétition pour le peptide IIINys de la chaîne alpha 1 télopeptidique aminoterminale du collagène de type III

Le peptide conjugué à la biotine dilué avec du tampon de revêtement ("coating") est adsorbé sur des plaques avidinées 2h à température ambiante. Après lavage, 50 µl d'antisérums dilués au titre optimal sont ajoutés à 50 µl de standard (peptide synthétique dilué dans du tampon) ou à 50 µl d'échantillons à doser et sont laissés à incuber pendant une nuit à 4°C. Après lavage, 100 µl d'anticorps secondaire couplés à la peroxydase dirigés contre les immunoglobulines de lapins sont ajoutés pendant une heure à température ambiante. Les puits sont lavés et 100 µl de substrat (TMB) sont ajoutés. La réaction est stoppée avec 100 µl d'H₂SO₄.

### 5. Analyse immunohistochimique de sections tissus synoviaux de patients atteints d'ostéoarthrite

La membrane synoviale de patients atteints d'arthrose a été prélevée lors d'une chirurgie de remplacement de genou. Les échantillons de la membrane synoviale ont été fixés et inclus dans de la paraffine selon les techniques usuelles de laboratoire. L'immunohistochimie a été réalisé sur des sections de 7 µm avec le diaminobenzidine (standard Envision^{™}+HRP protocol). Brièvement, les sections sont déparaffinée, les peroxydases endogènes sont bloquées et l'antigène est démasqué par une incubation de 2 heures à 60°C dans du tampon tris-EDTA (pH 9). Les sites non spécifiques sont bloqués avec de la caséine dans du TBS. Les sections sont par la suite mises à incuber durant une nuit à 4°C avec l'anticorps dirigés contre la séquence SEQ ID N°3 et l'anticorps non immun comme contrôle négatif (provenant du même lapin avant l'immunisation). Après des lavages soigneux, un anticorps anti-lapin Envision^{™} couplé à la HRP (DAKO) est ajouté aux sections et laissé à incubé durant 30 min à température ambiante. L'anticorps Envision^{™} en excès est éliminé par des lavages et la solution de substrat composé de diaminobenzidine (DAB+, DAKO) est ajoutée pour la réaction avec la peroxydase donnant une couleur brune. Le marquage au DAB est activé par le sulfate de cuivre (CuSO4) et le double marquage a été réalisé avec l'acide Haematoxyline de Mayers qui colore les noyaux des cellules.

### B - RESULTATS

### Exemple 1 : ELISA de compétition pour le peptide IIINys du N-télopeptide du collagène de type III.

Les méthodes de production des peptides, antisérums ainsi que la méthode ELISA de compétition sont décrites précédemment.

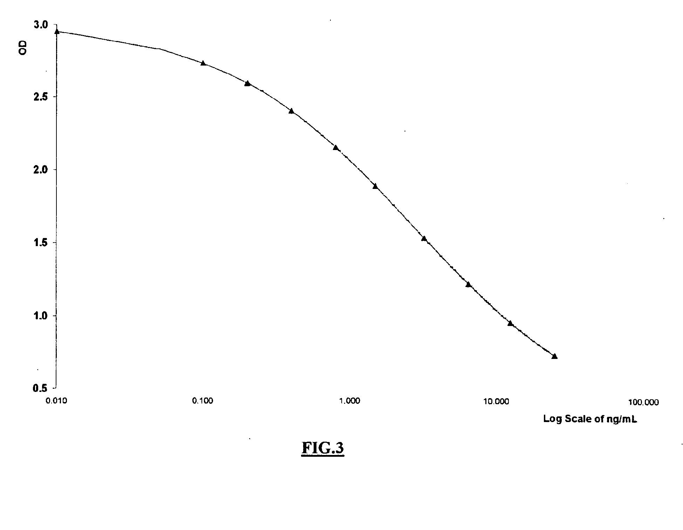

Une courbe standard ou de calibration a été obtenue sur un graphe semi log en dosant les 8 standards (de 0,2 ng/ml à 25 ng/ml) avec l'antisérum dirigé contre la séquence **SEQ ID N°2** sélectionné pour son titre (**Fig. 3**). La concentration en peptide de séquence **SEQ ID N°2** dans les échantillons est obtenue par extrapolation de la courbe de calibration (Densité Optique DO des 8 standards dosés en fonction du Log de leurs concentrations en ng/mL : *"Logscale of ng*/*mL").*

La détection limite de cet essai a été déterminée par le calcul de la moyenne de 32 déterminations du standard 0 auquel on retranche trois fois la déviation standard. La détection limite est de 0,16 ng/mL.

### Exemple 2 : Caractérisation de la spécificité de l'antisérum anti-IIINys

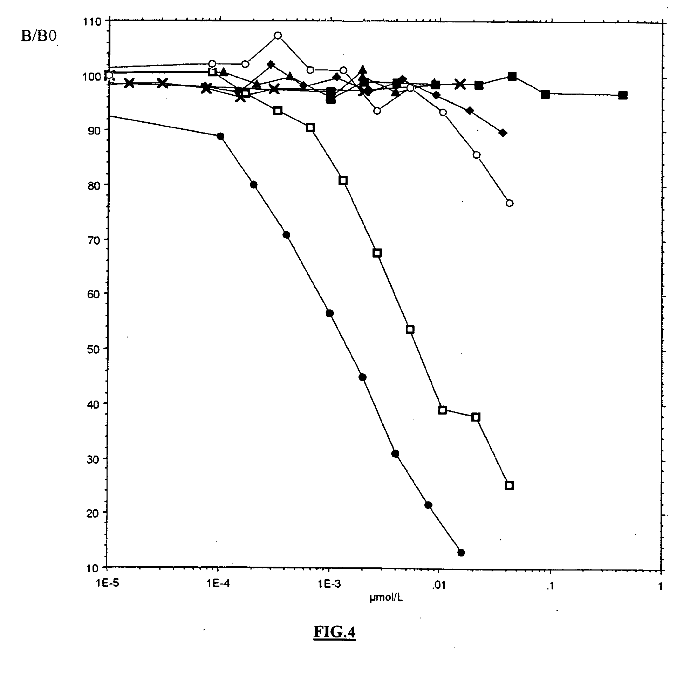

L'antisérum sélectionné a été testé vis-à-vis de différents peptides selon la méthode ELISA décrite précédemment afin de vérifier sa spécificité (**Fig.4**). Les peptides testés sont : le peptide IIINys de séquence SEQ ID N°2 (le peptide immunogène), le peptide de séquence SEQ ID N°3 (le peptide IIINys avec seulement la première tyrosine nitrosylée), le peptide de séquence SEQ ID N°4 (le peptide IIINys avec seulement la deuxième tyrosine nitrosylée), le peptide de séquence SEQ ID N°9 (le peptide IIINys avec un acide aminé supplémentaire du côté N-terminal), le peptide de séquence SEQ ID N°8 (le peptide IIINys avec ses tyrosines non nytrosylées), la 3-nitrotyrosine (**Fig.2**), avec la BSA nitrosylée de séquence SEQ ID N°11. Les peptides de séquence SEQ ID N°8 et 9 ainsi que la BSA nitrosylée et la 3-nitrotyrosine ne sont pas capables de rentrer en compétition avec le peptide IIINys (SEQ ID N°2) adsorbé sur la plaque. Par contre, l'antisérum reconnaît les peptides de séquence SEQ ID N°3 et 4 mais avec moins d'affinité que pour le peptide IIINys. Donc l'antisérum anti-IIINys est spécifique du site de coupure par la N-protéinase du côté N-terminal du N-télopeptide du collagène de type III et est plus spécifique également de la première nitrosylation.

### Exemple 3 : le niveau de IIINys sérique est augmenté chez les patients atteints de polyarthrites rhumatoïde en comparés à des patients sains.

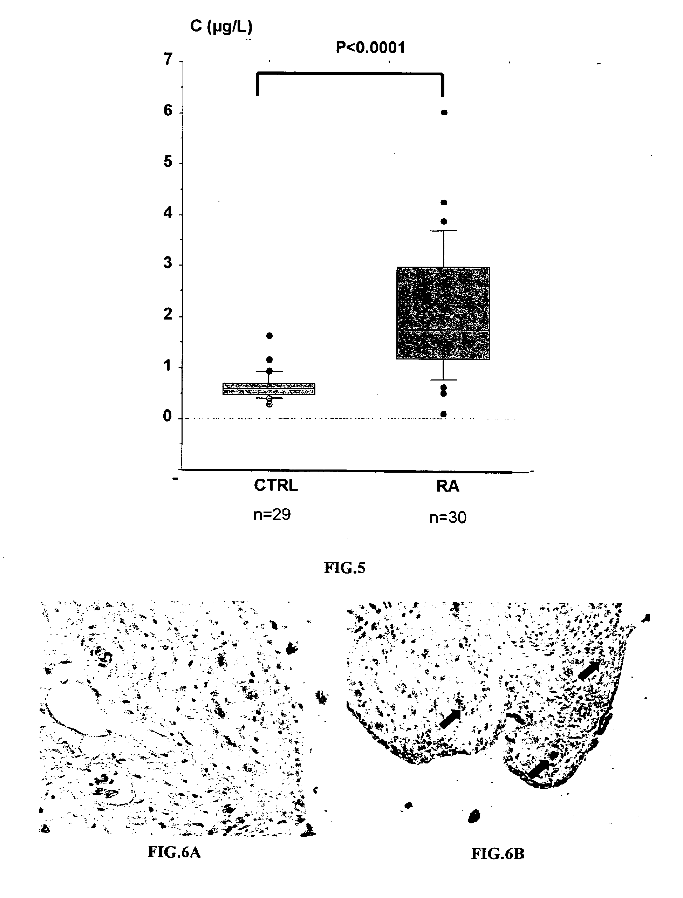

Des sérums de patients sains (n=29) et de patients atteints de polyarthrite rhumatoïde (n=30) ont été collectés et dosés en IIINys comme décrit dans le protocole. La concentration en IIINys (ng/ml) pour les différentes populations est présentée dans la **Fig. 5**. Les lignes horizontales représentent la médiane de IIINys (ng/ml) dans chaque groupe. Les valeurs de significativité de différence entre les groupes (P) sont représentées sur le graphe.

### Exemple 4 : Immunohistochimie sur tissus synoviaux de patients atteints d'arthrose.

L'immunohistochimie de tissus synoviaux de patients atteints d'arthrose du genou montre un marquage intense avec l'anticorps anti-IIINys dans la matrice extracellulaire, plus précisément autour des synoviocytes et à l'intérieur des macrophages (**Fig 6B**) et aucun marquage avec le sérum pré-immun (**Fig. 6A**), les flèches représentent l'espace pericellulaire des synoviocytes marqué par l'anticorps anti-IIINys.

## Revendications

1. Marqueur spécifique d'une dégradation oxydative de tissus contenant du collagène de type III, **caractérisé en ce qu'**il comprend au moins une séquence peptidique SP* de 6 à 25 acides aminés (AA), ladite séquence peptidique SP* comprenant la sous-séquence QYDSYD [SEQ ID N°1] dans laquelle au moins l'un des Y est nitrosylé* et où Q correspond non seulement à la glutamine conformément au code international mais aussi à l'acide pyroglutamique.

2. Marqueur selon la revendication 1, **caractérisé en ce que** les tissus visés comprennent le tissu synovial.

3. Marqueur selon la revendication 1 ou 2, **caractérisé en ce que** la séquence peptidique SP* est choisie :
→ dans le groupe de séquences suivantes:
• QY*DSY*DVKSG [SEQ ID N°2];
• QY*DSYDVKSG [SEQ ID N°3];
• QYDSY*DVKSG [SEQ ID N°4];
séquences dans lesquelles Y* correspond à un Y nitrosylé;
→ et, dans le groupe de séquences suivantes:
• QY*DSY*DVKS [SEQ ID N°5];
• QY*DSYDVKS [SEQ ID N°6];
• QYDSY*DVKS [SEQ ID N°7];
séquences dans lesquelles Y* correspond à un Y nitrosylé.

4. Marqueur selon l'une quelconque des revendications 1 à 3, **caractérisé en ce qu'**il comprend en outre la séquence peptidique SP non nitrosylée: QYDSYDVKSG [SEQ ID N°8].

5. Marqueur selon la revendication 4, **caractérisé en ce que** la séquence peptidique SP* ou SP, contenue dans une protéine P, est reliée par au moins un pontage à
- au moins une autre séquence peptidique SP* ou SP, contenue dans la même molécule de protéine P, ou
- à une autre séquence peptidique SP', contenue dans une autre molécule de protéine P, ou
- à au moins une autre séquence peptidique SP^{x}, contenue dans une protéine P^{x} différente de P.

6. Marqueur selon la revendication 5, **caractérisé en ce que** la protéine P est du collagène de type III, SP* ou SP étant contenue dans un télopeptide, et/ou **en ce que** la protéine P^{x} est du collagène de type différent du type III.

7. Séquence peptidique isolée du corps humain ou autrement produite par un procédé technique, **caractérisée en ce qu'**elle comprend de 6 à 25 acides aminés (AA), comprenant la sous-séquence QYDSYD [SEQ ID N°1] dans laquelle au moins l'un des Y est nitrosylé* et où Q correspond non seulement à la glutamine conformément au code international mais aussi à l'acide pyroglutamique.

8. Séquence peptidique isolée du corps humain ou autrement produite par un procédé technique, **caractérisée en ce qu'**elle est choisie dans le groupe de séquences suivantes:
• SP* telle que définie dans au moins l'une des revendications 1, 3, 5 et 6.

9. Utilisation d'au moins une séquence peptidique selon la revendication 7 ou 8, pour une application technique in vitro choisie dans le groupe comprenant:
• le diagnostic d'une pathologie qui s'accompagne d'une dégradation oxydative de tissus contenant du collagène de type III;
• le suivi d'une pathologie qui s'accompagne d'une dégradation oxydative de tissus contenant du collagène de type III;
• la détermination de l'efficacité d'un médicament adapté au traitement d'une pathologie qui s'accompagne d'une dégradation oxydative de tissus contenant du collagène de type III;
• ou la détermination de la toxicité d'un médicament adapté au traitement d'une pathologie qui s'accompagne d'une dégradation oxydative de tissus contenant du collagène de type III.

10. Utilisation selon la revendication 9, **caractérisée en ce que** la pathologie qui s'accompagne d'une dégradation oxydative de tissus contenant du collagène de type III, est une pathologie synoviale ou une autre pathologie ostéo-articulaire.

11. Méthode de diagnostic, de suivi, de pronostic d'une pathologie qui s'accompagne d'une dégradation oxydative de tissus contenant du collagène de type III, ou de détermination de l'efficacité ou de la toxicité d'un médicament adapté au traitement d'une pathologie qui s'accompagne d'une dégradation oxydative de tissus contenant du collagène de type III, **caractérisée en ce qu'**elle comprend les étapes essentielles suivantes:
a. analyse quantitative et/ou qualitative in vitro d'un échantillon biologique d'un individu, afin de détecter un marqueur spécifique d'une dégradation oxydative du tissu synovial selon l'une quelconque des revendications 1 à 6;
b. comparaison de la concentration en marqueur éventuellement détecté dans l'échantillon avec une concentration de référence correspondant à l'existence, à un stade déterminé, ou à l'absence chez l'individu, de la pathologie considérée.

12. Méthode selon la revendication 11, **caractérisée en ce que** la pathologie qui s'accompagne d'une dégradation oxydative de tissus contenant du collagène de type III est une pathologie synoviale ou une autre pathologie ostéo-articulaire.

13. Moyen pour le diagnostic, le suivi, le pronostic d'une pathologie qui s'accompagne d'une dégradation oxydative de tissus contenant du collagène de type III, ou pour la détermination de l'efficacité ou la toxicité d'un médicament adapté au traitement d'une pathologie qui s'accompagne d'une dégradation oxydative de tissus contenant du collagène de type III, **caractérisé en ce qu'**il comprend au moins un anticorps purifié apte à reconnaitre l'épitope compris dans la séquence peptidique SP* du marqueur selon l'une quelconque des revendications 1 à 6 et à s'associer avec la protéine porteuse dudit marqueur.

14. Anticorps purifié apte à reconnaître l'épitope compris dans la séquence peptidique SP* du marqueur selon l'une quelconque des revendications 1, 3, 5 et 6 ou la séquence selon la revendication 7 ou 8 et à s'associer de manière spécifique avec la protéine porteuse dudit marqueur ou de ladite séquence.

15. Kit de diagnostic, de suivi, de pronostic d'une pathologie qui s'accompagne d'une dégradation oxydative de tissus contenant du collagène de type III, ou de détermination de l'efficacité ou de la toxicité d'un médicament adapté au traitement d'une pathologie qui s'accompagne d'une dégradation oxydative de tissus contenant du collagène de type III, **caractérisé en ce qu'**il comprend au moins un moyen selon la revendication 13.

## Claims

1. Marker specific to an oxidative degradation of tissues containing type III collagen, **characterized in that** it comprises at least one peptide sequence SP* of 6 to 25 amino acids (AAs), said sequence SP* comprising the sub-sequence QYDSYD [SEQ ID No. 1] in which at least one of the Ys is nitrosylated* and where Q corresponds not only to glutamine in accordance with the international code but also to pyroglutamic acid.

2. Marker according to claim 1, **characterized in that** the tissues referred to comprise synovial tissue.

3. Marker according to claim 1 or 2, **characterized in that** the peptide sequence SP* is chosen:
→ from the following group of sequences:
■ QY*DSY*DVKSG [SEQ ID No. 2];
■ QY*DSYDVKSG [SEQ ID No. 3];
■ QYDSY*DVKSG [SEQ ID No. 4];
sequences in which Y* corresponds to a nitrosylated Y;
→ and, from the following group of sequences:
■ QY*DSY*DVKS [SEQ ID No. 5];
■ QY*DSYDVKS [SEQ ID No. 6];
■ QYDSY*DVKS [SEQ ID No. 7];
sequences in which Y* corresponds to a nitrosylated Y.

4. Marker according to any one of claims 1 to 3, **characterized in that** it further comprises the non-nitrosylated peptide sequence SP: QYDSYDVKSG [SEQ ID No. 8].

5. Marker according to claim 4, **characterized in that** the peptide sequence SP* or SP, contained in a protein P, is linked by at least one bridge to
- at least one other peptide sequence SP* or SP contained in the same molecule of protein P, or
- to another peptide sequence SP', contained in another molecule of protein P, or,
- to at least another peptide sequence SP^{x}, containing in protein P^{x}, different from P.

6. Marker according to claim 5, **characterized in that** the protein P is type III collagen, SP* or SP being contained in a telopeptide, and/or **in that** protein P^{x} is collagen of a type different from type III, preferably a type I collagen.

7. Peptide sequence isolated from the human body or otherwise produced by a technical process, **characterized in that** it comprises 6 to 25 amino acids (AAs), comprising the sub-sequence QYDSYD [SEQ ID No. 1] in which at least one of the Ys is nitrosylated* and where Q corresponds not only to glutamine in accordance with the international code but also to pyroglutamic acid.

8. Peptide sequence isolated from human body or otherwise produced by a technical process, **characterized in that** it is chosen from the following group of sequences:
■ SP* as defined in at least one of claims 1, 3, 5 and 6.

9. Use of at least one peptide sequence according to claim 7 or 8, for a technical application chosen from the group comprising:
■ the diagnosis of a pathology which is accompanied by an oxidative degradation of tissues containing type III collagen;
■ the monitoring of a pathology which is accompanied by an oxidative degradation of tissues containing type III collagen;
■ the determination of the effectiveness of a medicament suitable for the treatment of a pathology which is accompanied by an oxidative degradation of tissues containing type III collagen;
■ or the determination of the toxicity of a medicament suitable for the treatment of a pathology which is accompanied by an oxidative degradation of tissues containing type III collagen;

10. Use according to claim 9, **characterized in that** the pathology which is accompanied by an oxidative degradation of tissues containing type III collagen, is a synovial pathology or another osteoarticular pathology.

11. Method for the diagnosis, monitoring or prognosis of a pathology which is accompanied by an oxidative degradation of tissues containing type III collagen, **characterized in that** it comprises the following essential stages:
a. quantitative and/or qualitative *in vitro* analysis of a biological sample from an individual, in order to detect a marker specific to an oxidative degradation of the synovial tissue according to any one of claims 1 to 6;
b. comparison of the concentration of marker optionally detected in the sample with a reference concentration corresponding to the existence (at a determined stage) or to the absence in an individual, of the pathology considered.

12. Method according to claim 11, **characterized in that** the pathology which is accompanied by an oxidative degradation of tissues containing type III collagen is a synovial pathology or another osteoarticular pathology.

13. Means for the diagnosis, monitoring or prognosis of a pathology which is accompanied by an oxidative degradation of tissues containing type III collagen, or for determining the effectiveness or the toxicity of a medicament suitable for the treatment of a pathology which is accompanied by an oxidative degradation of tissues containing type III collagen, **characterized in that** it comprises at least one (purified) antibody capable of recognizing the epitope comprised in the peptide sequence SP* of the marker according to any one of claims 1 to 6 and of associating to the protein carrying said marker.

14. Purified antibody capable of recognizing the epitope comprised in the peptide sequence SP* of the marker according to any one of claims 1, 3, 5 and 6 or the sequence according to claim 7 or 8 and of associating to the protein carrying said marker or said sequence.

15. Kit for the diagnosis, monitoring or prognosis of a pathology which is accompanied by an oxidative degradation of tissues containing type III collagen, or for determining the effectiveness or toxicity of a medicament suitable for the treatment of a pathology which is accompanied by an oxidative degradation of tissues containing type III collagen, **characterized in that** it comprises at least one means according to claim 13.

## Patentansprüche

1. Spezifischer Marker für eine oxidative Degradation von Geweben, die Kollagen vom Typ III enthalten, **dadurch gekennzeichnet, dass** er mindestens eine Peptidsequenz SP* 6 bis 25 Aminosäuren (AA) aufweist, wobei die Peptidsequenz SP* die Subsequenz QYDSYD [SEQ ID Nr. 1] aufweist, wobei mindestens eines der Y nitrosiliert ist, und wo Q nicht nur dem Glutamin gemäß dem internationalen Code entspricht, aber auch der Pyroglutaminsäure.

2. Marker nach Anspruch 1, **dadurch gekennzeichnet, dass** die betroffenen Gewebe das synoviale Gewebe enthalten.

3. Marker nach Anspruch 1 oder 2, **dadurch gekennzeichnet, dass** die Peptidsequenz SP* ausgewählt ist:
→ aus der Gruppe der folgenden Sequenzen:
- QY*DSY*DVKSG [SEQ ID Nr. 2];
- QY*DSYDVKSG [SEQ ID Nr. 3];
- QYDSY*DVKSG [SEQ ID Nr. 4];
wobei in den Sequenzen Y* einem nitrosilierten Y entspricht;
→ und aus der Gruppe der folgenden Sequenzen:
- QY*DSY*DVKS [SEQ ID Nr. 5];
- QY*DSYDVKS [SEQ ID Nr. 6];
- QYDSY*DVKS [SEQ ID Nr. 7];
wobei in den Sequenzen Y* einem nitrosilierten Y entspricht.

4. Marker nach einem beliebigen der Ansprüche 1 bis 3, **dadurch gekennzeichnet, dass** er außerdem die nicht-nitrosilierte Peptidsequenz SP aufweist:
QYDSYDVKSG [SEQ ID Nr. 8].

5. Marker nach Anspruch 4, **dadurch gekennzeichnet, dass** die Peptidsequenz SP* oder SP, die in einem Protein P enthalten ist, über mindestens eine Brücke gebunden ist an
- mindestens eine andere Peptidsequenz SP* oder SP, die in dem gleichen Proteinmolekül P enthalten ist, oder
- eine andere Peptidsequenz SP', die in einem anderen Proteinmolekül P enthalten ist, oder
- mindestens eine andere Peptidsequenz SP^{x}, die in einem Protein P^{x}, das sich von P unterscheidet, enthalten ist.

6. Marker nach Anspruch 5, **dadurch gekennzeichnet, dass** das Protein P Kollagen vom Typ III ist, wobei SP* oder SP in einem Telopeptid enthalten sind, und/oder dass das Protein P^{x} Kollagen von einem anderen Typ als dem Typ III ist.

7. Peptidsequenz, die aus dem menschlichen Körper isoliert oder anderweitig durch ein technisches Verfahren erzeugt wurde, **dadurch gekennzeichnet, dass** sie 6 bis 25 Aminosäuren (AA) enthält, wobei die Peptidsequenz die Subsequenz QYDSYD [SEQ ID Nr. 1] enthält, in der mindestens eines der Y nitrosiliert* ist, und wo Q nicht nur dem Glutamin gemäß dem internationalen Code entspricht, aber auch der Pyroglutaminsäure.

8. Peptidsequenz, die aus dem menschlichen Körper isoliert oder anderweitig durch ein technisches Verfahren erzeugt wurde, **dadurch gekennzeichnet, dass** sie aus der Gruppe der folgenden Sequenzen ausgewählt ist:
- SP* gemäß der Definition, die in mindestens einem der Ansprüche 1, 3, 5 und 6 gegeben ist.

9. Verwendung mindestens einer Peptidsequenz nach Anspruch 7 oder 8 für eine technische in-vitro-Anwendung, die ausgewählt ist aus der Gruppe bestehend aus:
- die Diagnose einer Pathologie, die mit einer oxidativen Degradation von Geweben, die Kollagen vom Typ III enthalten, einhergeht;
- die Betreuung einer Pathologie, die mit einer oxidativen Degradation von Geweben, die Kollagen vom Typ III enthalten, einhergeht;
- die Bestimmung der Wirksamkeit eines Medikamentes, das für die Behandlung einer Pathologie geeignet ist, die mit einer oxidativen Degradation von Geweben, die Kollagen vom Typ III enthalten, einhergeht;
- oder die Bestimmung der Toxizität eines Medikamentes, das für die Behandlung einer Pathologie geeignet ist, die mit einer oxidativen Degradation von Geweben, die Kollagen vom Typ III enthalten, einhergeht.

10. Verwendung nach Anspruch 9, **dadurch gekennzeichnet, dass** die Pathologie, die mit einer oxidativen Degradation von Geweben, die Kollagen vom Typ III enthalten, einhergeht, eine synoviale Pathologie oder eine andere Knochen-Gelenk-Pathologie ist.

11. Verfahren zur Diagnose, zur Betreuung, zur Prognose einer Pathologie, die mit einer oxidativen Degradation von Geweben, die Kollagen vom Typ III enthalten, einhergeht, oder zur Bestimmung der Wirksamkeit oder der Toxizität eines Medikamentes, das zur Behandlung einer Pathologie geeignet ist, die mit einer oxidativen Degradation von Geweben, die Kollagen vom Typ III enthalten, einhergeht, **dadurch gekennzeichnet, dass** das Verfahren folgende wesentliche Schritte aufweist:
a. Quantitative Analyse und/oder qualitative Analyse in-vitro einer biologischen Probe eines Individuums, um einen spezifischen Marker einer oxidativen Degradation des synovialen Gewebes nach einem beliebigen der Ansprüche 1 bis 6 zu detektieren;
b. Vergleich der Konzentration des Markers, der gegebenenfalls in der Probe detektiert wurde, mit einer Vergleichskonzentration, die der Anwesenheit in einem bestimmten Stadium oder der Abwesenheit bei dem Individuum der genannten Pathologie entspricht.

12. Verfahren nach Anspruch 11, **dadurch gekennzeichnet, dass** die Pathologie, die mit einer oxidativen Degradation von Geweben, die Kollagen vom Typ III enthalten, einhergeht; eine synoviale Pathologie oder eine andere Knochen-Gelenk-Pathologie ist.

13. Mittel zur Diagnose, zur Betreuung, zur Prognose einer Pathologie, die mit einer oxidativen Degradation von Geweben, die Kollagen vom Typ III enthalten, einhergeht, oder zur Bestimmung der Wirksamkeit oder der Toxizität eines Medikamentes, das zur Behandlung einer Pathologie geeignet ist, die mit einer oxidativen Degradation von Geweben, die Kollagen vom Typ III enthalten, einhergeht, **dadurch gekennzeichnet, dass** das Mittel mindestens einen gereinigten Antikörper aufweist, der geeignet ist, das Epitop zu erkennen, das in der Peptidsequenz SP* des Markers nach einem beliebigen der Ansprüche 1 bis 6 enthalten ist, und sich mit dem Trägerprotein des Markers zu verbinden.

14. Gereinigter Antikörper, der geeignet ist, das Epitop zu erkennen, das in der Peptidsequenz SP* des Markers nach einem beliebigen der Ansprüche 1, 3, 5 und 6 oder in der Sequenz nach Anspruch 7 oder 8 enthalten ist, und sich auf spezifische Weise mit dem Trägerprotein des Markers oder der Sequenz zu verbinden.

15. Set zur Diagnose, zur Betreuung, zur Prognose einer Pathologie, die mit einer oxidativen Degradation von Geweben, die Kollagen vom Typ III enthalten, einhergeht, oder zur Bestimmung der Wirksamkeit oder der Toxizität eines Medikamentes, das zur Behandlung einer Pathologie geeignet ist, die mit einer oxidativen Degradation von Geweben, die Kollagen vom Typ III enthalten, einhergeht, **dadurch gekennzeichnet, dass** das Set mindestens ein Mittel nach Anspruch 13 aufweist.


### SEQUENCE LISTING

<110> SYNARC
<120> MARQUEUR SPECIFIQUE D'UNE DEGRADATION OXYDATIVE DE TISSUS CONTENANT DU COLLAGENE DE TYPE III, MOYENS ET METHODES, KITS DE DIAGNOTIC DE SUIVI OU DE PRONOSTIC DES PATHOLOGIES CIBLEES PAR CE MARQUEUR
<130> BFF060303
<160> 14
<170> PatentIn version 3.3
<210> 1
   <211> 6
   <212> PRT
   <213> Homo sapiens
<220>
   <221> Nitrosylation
   <222> (1)..(6)
   <223> Au moins l'un des Tyr est nitrosylé et où Gln correspond non seulement à la glutamine conformément au code international mais aussi à l'acide pyroglutamique
<400> 1
   Gln Tyr Asp Ser Tyr Asp
   1               5
<210> 2
   <211> 10
   <212> PRT
   <213> Homo sapiens
<220>
   <221> Nitrosylation
   <222> (1)..(10)
   <223> Xaa correspond à un Tyr Nitrosylé et Gln correspond non seulement a la glutamine conformément au code international mais aussi à l'acide pyroglutamique
<400> 2
   Gln Xaa Asp Ser Xaa Asp Val Lys Ser Gly
   1               5                   10
<210> 3
   <211> 10
   <212> PRT
   <213> Homo sapiens
<220>
   <221> Nitrosylation
   <222> (1)..(10)
   <223> xaa correspond à un Tyr Nitrosylé et Gln correspond non seulement à la glutamine conformément au code international mais aussi à l'acide pyroglutamique
<400> 3
   Gln Xaa Asp Ser Tyr Asp Val Lys Ser Gly
   1               5                   10
<210> 4
   <211> 10
   <212> PRT
   <213> Homo sapiens
<220>
   <221> Nitrosylation
   <222> (1)..(10)
   <223> Xaa correspond à un Tyr Nitrosylé et Gln correspond non seulement a la glutamine conformément au code international mais aussi à l'acide pyroglutamique
<400> 4
   Gln Tyr Asp Ser Xaa Asp Val Lys Ser Gly
   1               5                   10
<210> 5
   <211> 9
   <212> PRT
   <213> Homo sapiens
<220>
   <221> Nitrosylation
   <222> (1)..(9)
   <223> xaa correspond à un Tyr Nitrosylé et Gln correspond non seulement a la glutamine conformément au code international mais aussi à l'acide pyroglutamique
<400> 5
   Gln Xaa Asp Ser Xaa Asp Val Lys Ser
   1               5
<210> 6
   <211> 9
   <212> PRT
   <213> Homo sapiens
<220>
   <221> Nitrosylation
   <222> (1)..(9)
   <223> xaa correspond à un Tyr Nitrosylé et Gln correspond non seulement a la glutamine conformément au code international mais aussi à l'acide pyroglutamique
<400> 6
   Gln Xaa Asp Ser Tyr Asp Val Lys Ser
   1               5
<210> 7
   <211> 9
   <212> PRT
   <213> Homo sapiens
<220>
   <221> Nitrosylation
   <222> (1)..(9)
   <223> Xaa correspond à un Tyr Nitrosylé et Gln correspond non seulement a la glutamine conformément au code international mais aussi à l'acide pyroglutamique
<400> 7
   Gln Tyr Asp Ser Xaa Asp Val Lys Ser
   1               5
<210> 8
   <211> 10
   <212> PRT
   <213> Homo sapiens
<400> 8
   Gln Tyr Asp Ser Tyr Asp Val Lys Ser Gly
   1               5                   10
<210> 9
   <211> 11
   <212> PRT
   <213> Homo sapiens
<400> 9
   Pro Gln Tyr Asp Ser Tyr Asp Val Lys Ser Gly
   1               5                   10
<210> 10
   <211> 1466
   <212> PRT
   <213> Homo sapiens
<400> 10
   Met Met Ser Phe Val Gln Lys Gly Ser Trp Leu Leu Leu Ala Leu Leu
   1               5                   10                  15
   His Pro Thr Ile Ile Leu Ala Gln Gln Glu Ala Val Glu Gly Gly Cys
               20                  25                  30
   Ser His Leu Gly Gln Ser Tyr Ala Asp Arg Asp Val Trp Lys Pro Glu
           35                  40                  45
   Pro Cys Gln Ile Cys Val Cys Asp Ser Gly Ser Val Leu Cys Asp Asp
       50                  55                  60
   Ile Ile Cys Asp Asp Gln Glu Leu Asp Cys Pro Asn Pro Glu Ile Pro
   65                  70                  75                  80
   Phe Gly Glu Cys Cys Ala Val Cys Pro Gln Pro Pro Thr Ala Pro Thr
                   85                  90                  95
   Arg Pro Pro Asn Gly Gln Gly Pro Gln Gly Pro Lys Gly Asp Pro Gly
               100                 105                 110
   Pro Pro Gly Ile Pro Gly Arg Asn Gly Asp Pro Gly Ile Pro Gly Gln
           115                 120                 125
   Pro Gly Ser Pro Gly Ser Pro Gly Pro Pro Gly Ile Cys Glu Ser Cys
       130                 135                 140
   Pro Thr Gly Pro Gln Asn Tyr Ser Pro Gln Tyr Asp Ser Tyr Asp Val
   145                 150                 155                 160
   Lys Ser Gly Val Ala Val Gly Gly Leu Ala Gly Tyr Pro Gly Pro Ala
                   165                 170                 175
   Gly Pro Pro Gly Pro Pro Gly Pro Pro Gly Thr Ser Gly His Pro Gly
               180                 185                 190
   Ser Pro Gly Ser Pro Gly Tyr Gln Gly Pro Pro Gly Glu Pro Gly Gln
           195                 200                 205
   Ala Gly Pro Ser Gly Pro Pro Gly Pro Pro Gly Ala Ile Gly Pro Ser
       210                 215                 220
   Gly Pro Ala Gly Lys Asp Gly Glu Ser Gly Arg Pro Gly Arg Pro Gly
   225                 230                 235                 240
   Glu Arg Gly Leu Pro Gly Pro Pro Gly Ile Lys Gly Pro Ala Gly Ile
                   245                 250                 255
   Pro Gly Phe Pro Gly Met Lys Gly His Arg Gly Phe Asp Gly Arg Asn
               260                 265                 270
   Gly Glu Lys Gly Glu Thr Gly Ala Pro Gly Leu Lys Gly Glu Asn Gly
           275                 280                 285
   Leu Pro Gly Glu Asn Gly Ala Pro Gly Pro Met Gly Pro Arg Gly Ala
   290 295 300
   Pro Gly Glu Arg Gly Arg Pro Gly Leu Pro Gly Ala Ala Gly Ala Arg
   305 310 315 320
   Gly Asn Asp Gly Ala Arg Gly Ser Asp Gly Gln Pro Gly Pro Pro Gly
   325 330 335
   Pro Pro Gly Thr Ala Gly Phe Pro Gly Ser Pro Gly Ala Lys Gly Glu
   340 345 350
   Val Gly Pro Ala Gly Ser Pro Gly Ser Asn Gly Ala Pro Gly Gln Arg
   355 360 365
   Gly Glu Pro Gly Pro Gln Gly His Ala Gly Ala Gln Gly Pro Pro Gly
   370 375 380
   Pro Pro Gly Ile Asn Gly Ser Pro Gly Gly Lys Gly Glu Met Gly Pro
   385 390 395 400
   Ala Gly Ile Pro Gly Ala Pro Gly Leu Met Gly Ala Arg Gly Pro Pro
   405 410 415
   Gly Pro Ala Gly Ala Asn Gly Ala Pro Gly Leu Arg Gly Gly Ala Gly
   420 425 430
   Glu Pro Gly Lys Asn Gly Ala Lys Gly Glu Pro Gly Pro Arg Gly Glu
   435 440 445
   Arg Gly Glu Ala Gly Ile Pro Gly Val Pro Gly Ala Lys Gly Glu Asp
   450 455 460
   Gly Lys Asp Gly Ser Pro Gly Glu Pro Gly Ala Asn Gly Leu Pro Gly
   465 470 475 480
   Ala Ala Gly Glu Arg Gly Ala Pro Gly Phe Arg Gly Pro Ala Gly Pro
   485 490 495
   Asn Gly Ile Pro Gly Glu Lys Gly Pro Ala Gly Glu Arg Gly Ala Pro
   500 505 510
   Gly Pro Ala Gly Pro Arg Gly Ala Ala Gly Glu Pro Gly Arg Asp Gly
   515 520 525
   Val Pro Gly Gly Pro Gly Met Arg Gly Met Pro Gly Ser Pro Gly Gly
   530 535 540
   Pro Gly Ser Asp Gly Lys Pro Gly Pro Pro Gly Ser Gln Gly Glu Ser
   545                 550                 555                 560
   Gly Arg Pro Gly Pro Pro Gly Pro Ser Gly Pro Arg Gly Gln Pro Gly
                   565                 570                 575
   Val Met Gly Phe Pro Gly Pro Lys Gly Asn Asp Gly Ala Pro Gly Lys
               580                 585                 590
   Asn Gly Glu Arg Gly Gly Pro Gly Gly Pro Gly Pro Gln Gly Pro Pro
           595                 600                 605
   Gly Lys Asn Gly Glu Thr Gly Pro Gln Gly Pro Pro Gly Pro Thr Gly
       610                 615                 620
   Pro Gly Gly Asp Lys Gly Asp Thr Gly Pro Pro Gly Pro Gln Gly Leu
   625                 630                 635                 640
   Gln Gly Leu Pro Gly Thr Gly Gly Pro Pro Gly Glu Asn Gly Lys Pro
                   645                 650                 655
   Gly Glu Pro Gly Pro Lys Gly Asp Ala Gly Ala Pro Gly Ala Pro Gly
               660                 665                 670
   Gly Lys Gly Asp Ala Gly Ala Pro Gly Glu Arg Gly Pro Pro Gly Leu
           675                 680                 685
   Ala Gly Ala Pro Gly Leu Arg Gly Gly Ala Gly Pro Pro Gly Pro Glu
       690                 695                 700
   Gly Gly Lys Gly Ala Ala Gly Pro Pro Gly Pro Pro Gly Ala Ala Gly
   705                 710                 715                 720
   Thr Pro Gly Leu Gln Gly Met Pro Gly Glu Arg Gly Gly Leu Gly Ser
                   725                 730                 735
   Pro Gly Pro Lys Gly Asp Lys Gly Glu Pro Gly Gly Pro Gly Ala Asp
               740                 745                 750
   Gly Val Pro Gly Lys Asp Gly Pro Arg Gly Pro Thr Gly Pro Ile Gly
           755                 760                 765
   Pro Pro Gly Pro Ala Gly Gln Pro Gly Asp Lys Gly Glu Gly Gly Ala
       770                 775                 780
   Pro Gly Leu Pro Gly Ile Ala Gly Pro Arg Gly Ser Pro Gly Glu Arg
   785                 790                 795                 800
   Gly Glu Thr Gly Pro Pro Gly Pro Ala Gly Phe Pro Gly Ala Pro Gly
                   805                 810                 815
   Gln Asn Gly Glu Pro Gly Gly Lys Gly Glu Arg Gly Ala Pro Gly Glu
               820                 825                 830
   Lys Gly Glu Gly Gly Pro Pro Gly Val Ala Gly Pro Pro Gly Gly Ser
           835                 840                 845
   Gly Pro Ala Gly Pro Pro Gly Pro Gln Gly Val Lys Gly Glu Arg Gly
       850                 855                 860
   Ser Pro Gly Gly Pro Gly Ala Ala Gly Phe Pro Gly Ala Arg Gly Leu
   865                 870                 875                 880
   Pro Gly Pro Pro Gly Ser Asn Gly Asn Pro Gly Pro Pro Gly Pro Ser
                   885                 890                 895
   Gly Ser Pro Gly Lys Asp Gly Pro Pro Gly Pro Ala Gly Asn Thr Gly
               900                 905                 910
   Ala Pro Gly Ser Pro Gly Val Ser Gly Pro Lys Gly Asp Ala Gly Gln
           915                 920                 925
   Pro Gly Glu Lys Gly Ser Pro Gly Ala Gln Gly Pro Pro Gly Ala Pro
       930                 935                 940
   Gly Pro Leu Gly Ile Ala Gly Ile Thr Gly Ala Arg Gly Leu Ala Gly
   945                 950                 955                 960
   Pro Pro Gly Met Pro Gly Pro Arg Gly Ser Pro Gly Pro Gln Gly Val
                   965                 970                 975
   Lys Gly Glu Ser Gly Lys Pro Gly Ala Asn Gly Leu Ser Gly Glu Arg
               980                 985                 990
   Gly Pro Pro Gly Pro Gln Gly Leu  Pro Gly Leu Ala Gly  Thr Ala Gly
           995                 1000                 1005
   Glu Pro  Gly Arg Asp Gly Asn  Pro Gly Ser Asp Gly  Leu Pro Gly
       1010                 1015                 1020
   Arg Asp Gly Ser Pro Gly Gly Lys Gly Asp Arg Gly Glu Asn Gly
   1025 1030 1035
   Ser Pro Gly Ala Pro Gly Ala Pro Gly His Pro Gly Pro Pro Gly
   1040 1045 1050
   Pro Val Gly Pro Ala Gly Lys Ser Gly Asp Arg Gly Glu Ser Gly
   1055 1060 1065
   Pro Ala Gly Pro Ala Gly Ala Pro Gly Pro Ala Gly Ser Arg Gly
   1070 1075 1080
   Ala Pro Gly Pro Gln Gly Pro Arg Gly Asp Lys Gly Glu Thr Gly
   1085 1090 1095
   Glu Arg Gly Ala Ala Gly Ile Lys Gly His Arg Gly Phe Pro Gly
   1100 1105 1110
   Asn Pro Gly Ala Pro Gly Ser Pro Gly Pro Ala Gly Gln Gln Gly
   1115 1120 1125
   Ala Ile Gly Ser Pro Gly Pro Ala Gly Pro Arg Gly Pro Val Gly
   1130 1135 1140
   Pro Ser Gly Pro Pro Gly Lys Asp Gly Thr Ser Gly His Pro Gly
   1145 1150 1155
   Pro Ile Gly Pro Pro Gly Pro Arg Gly Asn Arg Gly Glu Arg Gly
   1160 1165 1170
   Ser Glu Gly Ser Pro Gly His Pro Gly Gln Pro Gly Pro Pro Gly
   1175 1180 1185
   Pro Pro Gly Ala Pro Gly Pro Cys Cys Gly Gly Val Gly Ala Ala
   1190 1195 1200
   Ala Ile Ala Gly Ile Gly Gly Glu Lys Ala Gly Gly Phe Ala Pro
   1205 1210 1215
   Tyr Tyr Gly Asp Glu Pro Met Asp Phe Lys Ile Asn Thr Asp Glu
   1220 1225 1230
   Ile Met Thr Ser Leu Lys Ser Val Asn Gly Gln Ile Glu Ser Leu
   1235 1240 1245
   Ile Ser Pro Asp Gly Ser Arg Lys Asn Pro Ala Arg Asn Cys Arg
   1250 1255 1260
   Asp Leu Lys Phe Cys His Pro Glu Leu Lys Ser Gly Glu Tyr Trp
   1265 1270 1275
   Val Asp Pro Asn Gln Gly Cys Lys Leu Asp Ala Ile Lys Val Phe
   1280 1285 1290
   Cys Asn Met Glu Thr Gly Glu Thr Cys Ile Ser Ala Asn Pro Leu
   1295 1300 1305
   Asn Val Pro Arg Lys His Trp Trp Thr Asp Ser Ser Ala Glu Lys
   1310 1315 1320
   Lys His Val Trp Phe Gly Glu Ser Met Asp Gly Gly Phe Gln Phe
   1325 1330 1335
   Ser Tyr Gly Asn Pro Glu Leu Pro Glu Asp Val Leu Asp Val Gln
   1340 1345 1350
   Leu Ala Phe Leu Arg Leu Leu Ser Ser Arg Ala Ser Gln Asn Ile
   1355 1360 1365
   Thr Tyr His Cys Lys Asn Ser Ile Ala Tyr Met Asp Gln Ala Ser
   1370 1375 1380
   Gly Asn Val Lys Lys Ala Leu Lys Leu Met Gly Ser Asn Glu Gly
   1385 1390 1395
   Glu Phe Lys Ala Glu Gly Asn Ser Lys Phe Thr Tyr Thr Val Leu
   1400 1405 1410
   Glu Asp Gly Cys Thr Lys His Thr Gly Glu Trp Ser Lys Thr Val
   1415 1420 1425
   Phe Glu Tyr Arg Thr Arg Lys Ala Val Arg Leu Pro Ile Val Asp
   1430 1435 1440
   Ile Ala Pro Tyr Asp Ile Gly Gly Pro Asp Gln Glu Phe Gly Val
   1445 1450 1455
   Asp Val Gly Pro Val Cys Phe Leu
   1460 1465
<210> 11
   <211> 607
   <212> PRT
   <213> Homo sapiens
<220>
   <221> SIGNAL
   <222> (1)..(18)
<220>
   <221> PROPEP
   <222> (19)..(24)
<220>
   <221> Nitrosylation
   <222> (24)..(607)
   <223> Xaa est la 3-Nitrotyrosine
<400> 11
   Met Lys Trp Val Thr Phe Ile Ser Leu Leu Leu Leu Phe Ser Ser Ala
   1               5                   10                  15
   Tyr Ser Arg Gly Val Phe Arg Arg Asp Thr His Lys Ser Glu Ile Ala
               20                  25                  30
   His Arg Phe Lys Asp Leu Gly Glu Glu His Phe Lys Gly Leu Val Leu
           35                  40                  45
   Ile Ala Phe Ser Gln Xaa Leu Gln Gln Cys Pro Phe Asp Glu His Val
       50                  55                  60
   Lys Leu Val Asn Glu Leu Thr Glu Phe Ala Lys Thr Cys Val Ala Asp
   65                  70                  75                  80
   Glu Ser His Ala Gly Cys Glu Lys Ser Leu His Thr Leu Phe Gly Asp
                   85                  90                  95
   Glu Leu Cys Lys Val Ala Ser Leu Arg Glu Thr Xaa Gly Asp Met Ala
               100                 105                 110
   Asp Cys Cys Glu Lys Gln Glu Pro Glu Arg Asn Glu Cys Phe Leu Ser
           115                 120                 125
   His Lys Asp Asp Ser Pro Asp Leu Pro Lys Leu Lys Pro Asp Pro Asn
       130                 135                 140
   Thr Leu Cys Asp Glu Phe Lys Ala Asp Glu Lys Lys Phe Trp Gly Lys
   145                 150                 155                 160
   Xaa Leu Xaa Glu Ile Ala Arg Arg His Pro Xaa Phe Xaa Ala Pro Glu
                   165                 170                 175
   Leu Leu Xaa Xaa Ala Asn Lys Xaa Asn Gly Val Phe Gln Glu Cys Cys
           180                 185                 190
   Gln Ala Glu Asp Lys Gly Ala Cys Leu Leu Pro Lys Ile Glu Thr Met
       195                 200                 205
   Arg Glu Lys Val Leu Ala Ser Ser Ala Arg Gln Arg Leu Arg Cys Ala
       210                 215                 220
   Ser Ile Gln Lys Phe Gly Glu Arg Ala Leu Lys Ala Trp Ser Val Ala
   225                 230                 235                 240
   Arg Leu Ser Gln Lys Phe Pro Lys Ala Glu Phe Val Glu Val Thr Lys
                   245                 250                 255
   Leu Val Thr Asp Leu Thr Lys Val His Lys Glu Cys Cys His Gly Asp
               260                 265                 270
   Leu Leu Glu Cys Ala Asp Asp Arg Ala Asp Leu Ala Lys Xaa Ile Cys
           275                 280                 285
   Asp Asn Gln Asp Thr Ile Ser Ser Lys Leu Lys Glu Cys Cys Asp Lys
       290                 295                 300
   Pro Leu Leu Glu Lys Ser His Cys Ile Ala Glu Val Glu Lys Asp Ala
   305                 310                 315                 320
   Ile Pro Glu Asn Leu Pro Pro Leu Thr Ala Asp Phe Ala Glu Asp Lys
                   325                 330                 335
   Asp Val Cys Lys Asn Xaa Gln Glu Ala Lys Asp Ala Phe Leu Gly Ser
               340                 345                 350
   Phe Leu Xaa Glu Xaa Ser Arg Arg His Pro Glu Xaa Ala Val Ser Val
           355                 360                 365
   Leu Leu Arg Leu Ala Lys Glu Xaa Glu Ala Thr Leu Glu Glu Cys Cys
       370                 375                 380
   Ala Lys Asp Asp Pro His Ala Cys Xaa Ser Thr Val Phe Asp Lys Leu
   385                 390                 395                 400
   Lys His Leu Val Asp Glu Pro Gln Asn Leu Ile Lys Gln Asn Cys Asp
                   405                 410                 415
   Gln Phe Glu Lys Leu Gly Glu Xaa Gly Phe Gln Asn Ala Leu Ile Val
             420                 425                 430
   Arg Xaa Thr Arg Lys Val Pro Gln Val Ser Thr Pro Thr Leu Val Glu
         435                 440                 445
   Val Ser Arg Ser Leu Gly Lys Val Gly Thr Arg Cys Cys Thr Lys Pro
       450                 455                 460
   Glu Ser Glu Arg Met Pro Cys Thr Glu Asp Xaa Leu Ser Leu Ile Leu
   465                 470                 475                 480
   Asn Arg Leu Cys Val Leu His Glu Lys Thr Pro Val Ser Glu Lys Val
                   485                 490                 495
   Thr Lys Cys Cys Thr Glu Ser Leu Val Asn Arg Arg Pro Cys Phe Ser
               500                 505                 510
   Ala Leu Thr Pro Asp Glu Thr Tyr Val Pro Lys Ala Phe Asp Glu Lys
           515                 520                 525
   Leu Phe Thr Phe His Ala Asp Ile Cys Thr Leu Pro Asp Thr Glu Lys
       530                 535                 540
   Gln Ile Lys Lys Gln Thr Ala Leu Val Glu Leu Leu Lys His Lys Pro
   545                 550                 555                 560
   Lys Ala Thr Glu Glu Gln Leu Lys Thr Val Met Glu Asn Phe Val Ala
                   565                 570                 575
   Phe Val Asp Lys Cys Cys Ala Ala Asp Asp Lys Glu Ala Cys Phe Ala
               580                 585                 590
   Val Glu Gly Pro Lys Leu Val Val Ser Thr Gln Thr Ala Leu Ala
           595                 600                 605
<210> 12
   <211> 6
   <212> PRT
   <213> Homo sapiens
<400> 12
   Asp Val Lys Ser Gly Val
   1               5
<210> 13
   <211> 9
   <212> PRT
   <213> Homo sapiens
<220>
   <221> Nitrosylation
   <222> (1)..(9)
   <223> Xaa correspond à un Tyr Nitrosylé
<400> 13
   His Arg Gly Xaa Pro Gly Leu Asp Gly
   1               5
<210> 14
   <211> 6
   <212> PRT
   <213> Homo sapiens
<220>
   <221> Nitrosylation
   <222> (1)..(6)
   <223> Xaa correspond à un Tyr Nitrosylé
<400> 14
   Leu Gln Xaa Met Arg Ala
   1               5